# EUROPEAN PATENT SPECIFICATION

(11) **EP 3 522 717 B1**
(45) Date of publication and mention of the grant of the patent: **24.01.2024**
(21) Application number: 17777607.7
(22) Date of filing: 06.10.2017
(51) Int. Cl.: A01N 59/00, A61K 33/40, A61K 8/22, A01P 1/00, A61K 31/43, A61K 31/7004, A61K 45/06, A61K 8/60, A61K 8/66, A61K 9/00, A61K 9/107, A61P 1/02, A61K 38/44, A61P 11/02, A61P 17/00, A61P 17/02, A61P 31/00, A61P 31/04, A61P 31/16, A61P 43/00, A61Q 17/00

(54) **ANTIMICROBIAL COMPOSITIONS**
ANTIMIKROBIELLE ZUSAMMENSETZUNGEN
COMPOSITIONS ANTIMICROBIENNES

(30) Priority: 06.10.2016 EP 16192639; 06.10.2016 EP 16192635; 23.01.2017 EP 17152718; 23.01.2017 EP 17152715; 25.04.2017 EP 17168085; 25.04.2017 EP 17168084
(43) Date of publication of application: 14.08.2019
(73) Proprietor: Matoke Holdings Limited, Abingdon, Oxfordshire OX13 5HR (GB)
(72) Inventor: KERSHAW, David, Abingdon Oxfordshire OX13 5HR (GB); DRYDEN, Matthew, Abingdon Oxfordshire OX13 5HR (GB); STAPLES, Ian, Abingdon Oxfordshire OX13 5HR (GB); COX, Sophie Constance, Birmingham West Midlands B15 2TT (GB); HALL, Thomas Jon, Birmingham West Midlands B15 2TT (GB); GROVER, Liam Michael, Birmingham West Midlands B15 2TT (GB); SALIB, Rami, Eastleigh Hampshire SO50 7FD (GB)
(74) Representative: Carridge, Andrew Edward
(86) International application number: PCT/EP2017/075554
(87) International publication number: WO 2018/065608

(56) References cited:
- EP-A1- 0 518 445
- WO-A1-2008/041218
- WO-A1-2009/118379
- WO-A1-2012/030231
- WO-A1-2015/166197
- WO-A1-2016/083798
- WO-A2-2007/045252
- US-A- 4 537 764
- US-A- 4 576 817
- US-A- 4 617 190
- US-A1- 2010 098 645
- S Bogdanov: "Characterisation of Antibacterial Substances in Honey", Lebensmittel Wissenschaft und Technologie, 1 January 1984 (1984-01-01), pages 74-76, XP055418659, Retrieved from the Internet: URL:http://www.bee-hexagon.net/files/file/ fileE/Honey/Bogdanov_LWT_1984_sb.pdf [retrieved on 2017-10-24]
- TIINA M ET AL: "Antibacterial effect of the glucose oxidase-glucose system on food-poisoning organisms", INTERNATIONAL JOURNAL OF FOOD MICROBIOLOGY, ELSEVIER BV, NL, vol. 8, no. 2, 1 May 1989 (1989-05-01), pages 165-174, XP023698025, ISSN: 0168-1605, DOI: 10.1016/0168-1605(89)90071-8 [retrieved on 1989-05-01]
- Simona Sagona ET AL: "Preliminary evaluation of glucose oxidase and its products in vitro antimicrobial activities on Paenibacillus larvae ATCC9545 vegetative form", Bulletin of Insectology, 1 January 2015 (2015-01-01), pages 233-237, XP55418305, Retrieved from the Internet: URL:http://www.bulletinofinsectology.org/p dfarticles/vol68-2015-233-237sagona.pdf
- .: "Biovert(TM) Enzyme & Substrate (patented) A Two-Part Natural Protection System", , 1 December 2014 (2014-12-01), pages 1-2, XP55418502, Retrieved from the Internet: URL:http://dewolfchem.com/wp-content/uploa ds/2014/12/2014_12_Biovert_SLS_lowres.pdf [retrieved on 2017-10-24]

## Description

This invention relates to compositions for generating antimicrobial activity, particularly compositions that are able to generate hydrogen peroxide.

Honey has been used for treatment of microbial infections since ancient times. In recent years there has been a resurgence of interest in the therapeutic efficacy of honey, particularly in the area of wound healing. Clinical trials have shown that honey is an effective broad-spectrum antimicrobial agent which is effective against common wound-infecting organisms, such as *Pseudomonas aeruginosa, Staphylococcus aureus, Candida albicans and Escherichia coli,* and is effective against antibiotic-resistant strains of bacteria. As a natural product, honey also offers an attractive alternative to drug-based treatments.

Many different types of honey have antimicrobial activity. This activity is attributed largely to osmolarity, pH, hydrogen peroxide production and the presence of phytochemical components.

The applicant has appreciated that the antimicrobial effects of honey can be greatly enhanced and controlled by adding glucose oxidase to honey, and that compositions comprising honey and added glucose oxidase are applicable in the treatment of a number of infections, and notably in the treatment of infections caused by biofilms (see WO 2015/166197, WO 2016/083798 and WO 2016/124926).

However, because honey is a natural product, its composition can vary greatly depending on its source. For example, the difference in antimicrobial potency among honeys can be more than one hundred-fold, depending on the geographical, seasonal and botanical source of the honey, as well as the harvesting, processing and storage conditions. Consequently, the antimicrobial efficacy may also vary depending on the type of honey used. Furthermore, honey may also contain other components, such as allergens e.g. trace amounts of pollen, which may cause adverse reactions when applied to certain subjects and make it unsuitable for certain pharmaceutical applications.

Honey may require processing such that it is in a suitable form for application to subjects, which can add cost and complexity to the production process. Such processing may include creaming or pasteurisation.

Consequently, there is a desire to provide improved compositions which provide some of the antimicrobial benefits provided by honey, but which also overcome some of its disadvantages. There is also a desire to provide compositions that are able to retain antimicrobial efficacy over an extended period of time.

WO2008/041218A1 discloses a storage-stable antimicrobial and immunostimulatory system comprising an oxidoreductase enzyme, a substrate for the oxidoreductase enzyme and hydrogen peroxide in an aqueous solution wherein the substrate for the oxidoreductase enzyme is present up to 90% by weight and water is present up to 20% by weight based on the weight of the total composition; the system has a pH from approximately 4 to 8; and the system provides a two-stage hydrogen peroxide release.

WO2015/166197A1 discloses storage-stable compositions comprising an enzyme that is able to convert a substrate to release hydrogen peroxide, and an unrefined natural substance, such as a honey, that includes a substrate for the enzyme. In certain examples, the enzyme is a purified enzyme. In other examples, the substrate lacks catalase activity, and the enzyme is additional to any enzyme activity able to convert the substrate to release hydrogen peroxide that may be present in the unrefined natural substance. The storage-stable compositions do not include sufficient free water to allow the enzyme to convert the substrate.

In a broad sense, the invention provides a composition comprising: an enzyme that is able to convert a substrate to release hydrogen peroxide; and a substrate for the enzyme.

According to the invention, there is provided a liquid or gel composition for generating antimicrobial activity comprising: a purified enzyme that is able to convert a substrate to release hydrogen peroxide; a purified substrate for the enzyme; a solute in the form of a sugar having a solubility of at least 100g/100g water at 20°C and 1 atm, preferably at least 300g/100g water; and a polymer. The composition has a water activity of less than 0.6 and substantially no hydrogen peroxide and does not comprise honey.

Surprisingly, the applicant has found that compositions comprising a purified enzyme and a purified substrate for the enzyme can be more effective at killing microorganisms than known honey-based compositions that can generate hydrogen peroxide.

References herein to "enzyme" refer to one or more enzyme. For example, in some embodiments, compositions of the invention may comprise a plurality of enzymes that are able to convert a substrate to release hydrogen peroxide. In some embodiments, compositions of the invention may comprise only one enzyme that is able to convert a substrate to release hydrogen peroxide.

The term "purified enzyme" is used herein to include an enzyme preparation in which the enzyme has been separated from at least some of the impurities originally present when the enzyme was produced. Preferably impurities that have been removed or reduced include those that would otherwise interfere with the ability of the enzyme to convert the substrate to release hydrogen peroxide.

It may not always be necessary or desirable that the purified enzyme is at a high level of purity provided that the enzyme is able to convert the substrate to release hydrogen peroxide. In some circumstances, it may be desirable to use a relatively crude enzyme preparation. Examples of suitable purity levels include at least 10%, 20%, 30%, 40%, 50%, 60%, 70%, 80%, or 90% pure. Preferably, the enzyme is at least 90% pure. Even more preferably, the enzyme is at least 99% pure.

The enzyme may have been produced by recombinant or non-recombinant means, and may be a recombinant or non-recombinant enzyme. The enzyme may be purified from a microbial source, preferably from a non-genetically modified microbe.

The level of purity of the enzyme may be selected as appropriate depending on the intended use of the composition. For medical use, a medical grade or medical device grade of purity should be used. For pharmaceutical use, a pharmaceutical grade of purity should be used.

Compositions of the invention may comprise sufficient enzyme and substrate to provide for sustained release of hydrogen peroxide at a specific level or concentration.

Compositions of the invention may comprise sufficient enzyme and substrate to provide for sustained release of hydrogen peroxide at a level of less than 2 mmol/litre for a period of at least twenty four hours, optionally following dilution of the composition.

Compositions of the invention may comprise sufficient enzyme and substrate to provide for sustained release of at least 0.02, 0.03, 0.04, 0.05, 0.1, 0.2, 0.3, 0.4, 0.5, 1 or 1.5 mmol/litre hydrogen peroxide for a period of at least 24 hours, more preferably 48 hours.

So, in some embodiments, compositions of the invention may comprise sufficient enzyme and substrate to provide for sustained release of 0.1 to 2 mmol/litre hydrogen peroxide for a period of at least 24 hours, more preferably 48 hours.

For example, in some embodiments, compositions of the invention may provide for sustained release of hydrogen peroxide at a concentration of at least 2 ppm, at least 5 ppm, at least 10 ppm, at least 20 ppm or at least 50 ppm. In preferred embodiments, the level may be at least 2 ppm. In some embodiments, the concentration may be, at the most, 500 ppm, 200 ppm, 100 ppm, 50 ppm, 20 ppm or 10 ppm. In preferred embodiments, the level may be 20 ppm or less. In even more preferred embodiments, the level may be 10 ppm or less. For example, the concentration may be 10 to 500 ppm, 20 to 200 ppm or 50 to 100 ppm, 2 to 50 ppm, 2 to 20 ppm or 5 to 10 ppm. If the composition does not comprise sufficient free water to allow the enzyme to convert the substrate (e.g. if the composition is a dry or dried composition), hydrogen peroxide production may only occur once it has been diluted by water and there is sufficient free water to allow the enzyme to convert the substrate. Addition of water may thus initiate hydrogen peroxide production. Compositions, of the invention may provide for sustained release of hydrogen peroxide for at least 1 hour, at least 12 hours, at least 24 hours, at least 2 days, or at least 4 days. Preferably, the level of hydrogen peroxide is sustained for at least 4 days. In preferred embodiments, the level of hydrogen peroxide is sustained at 10 to 500 ppm for at least 1 hour, at least 12 hours, at least 24 hours, at least 2 days, or at least 4 days. In other embodiments, the level of hydrogen peroxide is sustained at 50 to 100 ppm for at least 1 hour, at least 12 hours, at least 24 hours, at least 2 days, or at least 4 days. In other embodiments, the level of hydrogen peroxide is sustained at 2 to 50 ppm for at least 12 hours, at least 24 hours, at least 2 days, or at least 4 days. In other embodiments, the level of hydrogen peroxide is sustained at 5 to 10 ppm for at least 12 hours, at least 24 hours, at least 2 days, or at least 4 days. In some embodiments, compositions of the invention may provide for sustained release of 2 to 500 ppm hydrogen peroxide for at least 24 hours.

Compositions of the invention may comprise 25 to 2000 ppm of the enzyme, for example 50 to 1000 ppm of the enzyme. Compositions of the invention may comprise 750 to 2000 ppm of the enzyme. Compositions of the invention may comprise greater than 500 ppm of the enzyme. Compositions of the invention may comprise 250 to 1500 ppm of the enzyme.

The enzyme activity (for example, the glucose oxidase activity) may range, for example, from 1-400 IU/mg, or 1-300 IU/mg, for example 250-280 IU/mg. The amount of enzyme used is likely to depend on several factors, including the desired use of the composition, the desired level of hydrogen peroxide release, and the desired length of time for hydrogen peroxide release. A suitable amount of enzyme can readily be determined by a person of ordinary skill in the art, if necessary using a well diffusion assay, to determine the extent of hydrogen peroxide release for different amounts of enzyme. Suitable amounts of enzyme (such as glucose oxidase) may be from 0.0001% to 0.5% w/w of the composition. The amount of enzyme used may be selected so as to produce a composition for generating antimicrobial activity that is equivalent to a selected phenol standard (for example a 10%, 20%, or 30% phenol standard).

Compositions of the invention may comprise at least 1 unit, and preferably up to 1500 units, of the enzyme per gram of the composition. A "unit" is defined herein as the amount of enzyme (e.g. glucose oxidase) causing the oxidation of 1 micromole of substrate (e.g. glucose) per minute at 25 degrees centigrade at pH 7.0.

In some embodiments, a composition according to the invention comprises more than 15 units, for example at least 30 units, at least 50 units, or at least 100 units, and suitably less than 685 units, for example 100-500 units, of enzyme (e.g. glucose oxidase) per gram of the composition.

In other embodiments of the invention, a composition according to the invention comprises at least 500 units, for example 500-1000 units, or 685-1000 units, of enzyme (e.g. glucose oxidase) per gram of the composition.

References herein to "substrate" or "precursor-substrate" refer to one or more substrate or one or more precursor-substrate. For example, in some embodiments, compositions of the invention may comprise a plurality of substrates. In some embodiments, compositions of the invention may comprise only one substrate.

The term "purified substrate" or "purified precursor-substrate" is used herein to include a substrate or precursor-substrate preparation in which the substrate or precursor-substrate has been separated from at least some of the impurities originally present when the substrate or precursor-substrate was obtained or produced. The purified substrate or precursor-substrate may be obtained from a natural source or may be synthetically produced. The purified substrate may be a processed, extracted, or refined substrate or precursor-substrate (i.e. a substrate or precursor-substrate in which impurities or unwanted elements have been removed by processing).

It may not always be necessary or desirable that the purified substrate or precursor-substrate is at a high level of purity provided that the enzyme is able to convert the substrate to release hydrogen peroxide. In some circumstances, it may be desirable to use a relatively crude substrate or precursor-substrate preparation. Examples of suitable purity levels include at least 10%, 20%, 30%, 40%, 50%, 60%, 70%, 80%, 90%, 95%, or 99% pure. Preferably the purity level is at least 90%. Even more preferably, the purity is at least 99%. However, in some embodiments, it may be desirable that the purified substrate or precursor-substrate is a medical grade, medical device grade, or pharmaceutical grade substrate.

In particular embodiments, the purified substrate or precursor-substrate is or comprises a purified sugar. The term "sugar" is used herein to refer to a carbohydrate with the general formula Cₘ(H₂O)ₙ. The purified sugar may be obtained from a natural source (for example a processed, extracted, or refined natural sugar), or be synthetically produced. The purified sugar may be at least 10%, 20%, 30%, 40%, 50%, 60%, 70%, 80%, 90%, 95%, or 99% pure. Preferably the purity level is at least 90%. Even more preferably, the purity is at least 99%. The purified sugar may be a medical grade, medical device grade, or pharmaceutical grade sugar. The sugar may include, for example purified D-glucose, hexose, or D-galactose. For example, the purified sugar may be medical grade, medical device grade, or pharmaceutical grade D-glucose, hexose, or D-galactose.

In particular embodiments, the enzyme and the substrate are purified, for example purified glucose oxidase and purified D-glucose, suitably medical grade, medical device grade, or pharmaceutical grade glucose oxidase and D-glucose.

Preferably, the compositions of the invention are storage stable. Compositions of the invention do not include sufficient free water to allow the enzyme to convert the substrate Preferably, compositions of the invention do not comprise sufficient free water to allow the precursor-substrate to be converted to the substrate.

The composition comprises a purified enzyme that is able to convert a substrate to release hydrogen peroxide; and a purified substrate for the enzyme, wherein the composition does not include sufficient free water to allow the enzyme to convert the substrate.

The term "storage-stable" is used herein to mean that the composition can be stored at ambient temperature for at least several days, preferably at least a week, more preferably at least one or two months, whilst retaining the ability to generate antimicrobial activity following dilution of the composition. A preferred storage temperature is below 37°C, preferably 20-25°C. Preferably compositions are stored away from exposure to light.

Hydrogen peroxide is generally unstable at ambient temperature. The lack of sufficient free water in a storage-stable composition of the invention prevents the enzyme converting the substrate to release hydrogen peroxide, and thus helps to maintain the stability of the composition for extended periods at ambient temperature. A storage-stable composition of the invention may include some water provided that there is not sufficient free water to allow the enzyme to convert the substrate. Suitable amounts of water will vary depending on the precise components of the composition. However, typically, a storage-stable composition of the invention preferably comprises less than 20% (by weight) total water content, for example, 10%-19%, water.

Compositions of the invention may comprise 12% or less (by weight) of water.

Compositions of the invention may comprise 10% or less (by weight) of water.

Compositions of the invention may comprise 5% or less (by weight) of water.

Compositions of the invention may comprise 3% or less (by weight) of water.

In any case, the water activity in the composition according to the invention has to be less than 0.6.

Compositions of the invention comprise substantially no hydrogen peroxide, or no detectable hydrogen peroxide. For example, hydrogen peroxide is preferably not detectable using a hydrogen peroxide test strip, such as a Quantofix^{®} peroxide test stick (Sigma Aldrich, UK). For example, hydrogen peroxide may present at a level less than 1 ppm or at a level less than 0.5 ppm. Hydrogen peroxide may be at a level less than 0.1 ppm.

Compositions of the invention comprise an additional component in the form of a solute that is a sugar. References herein to "solute" refer to one or more solute. For example, in some embodiments, compositions of the invention may comprise a plurality of solutes. In some embodiments, the composition may only comprise one solute. According to the invention the solute has an aqueous solubility of at least 100 g per 100 g water.

The solute may be distinct from the substrate, or precursor-substrate, or in some examples, the substrate or precursor-substrate may be the same as the solute. For example, the composition may comprise fructose and fructose oxidase: the fructose being both the solute and the substrate for enzyme. In another example, the substrate may be glucose and the solute may be fructose.

The solute is preferably purified, meaning that the solute has been separated from at least some of the impurities originally present when the solute was obtained or produced. The purified solute may be obtained from a natural source or may be synthetically produced. The purified solute may be a processed, extracted, or refined substrate (i.e. a solute in which impurities or unwanted elements have been removed by processing).

It may not always be necessary or desirable that the purified solute is at a high level of purity. In some circumstances, it may be desirable to use a relatively crude solute preparation. Examples of suitable purity levels include at least 10%, 20%, 30%, 40%, 50%, 60%, 70%, 80%, 90%, 95%, or 99% pure. Preferably, the purity level is at least 90%. However, in some embodiments, it may be desirable that the purified solute is a medical grade, medical device grade, or pharmaceutical grade solute.

According to the invention, the solute is a sugar that has a solubility of at least 100 g/100 g water. Suitable sugars include oligosaccharides, disaccharides or monosaccharides. Preferably, the sugar is a disaccharide or a monosaccharide. In particularly preferred embodiments, the sugar is a monosaccharide. Suitable sugars may include fructose, sucrose or maltose that have a solubility of more than 100 g/100 g water. In a particularly preferred embodiment, the sugar is fructose.

Compositions of the invention may comprise two or more solutes, as described herein. So, compositions of the invention may comprise two or more sugars. The composition may comprise a maximum of two solutes, i.e. two sugars; or a maximum of three solutes, i.e. three sugars.

For instance, a composition of the invention may comprise glucose, fructose and sucrose, whereby glucose is not a solute with an aqueous solubility of at least 100 g/100 g of water.

The composition may comprise: a purified enzyme that is able to convert a substrate to release hydrogen peroxide; a purified substrate for the enzyme; and at least two sugars or sugar derivatives, wherein the composition does not include sufficient free water to allow the enzyme to convert the substrate.

The solute has a high solubility in water, that is a solubility which is greater than glucose. Glucose has a solubility of 90g/100g water at 20°C and 1 atm. According to the invention, the solute has a solubility greater than or equal to 100g/100g water at 20°C and 1 atm, in a more preferred embodiment, the solute has a solubility greater than or equal to 200g/100g water at 20°C and 1 atm, in an even more preferred embodiment, the solute has a solubility greater than 300g/100g water at 20°C and 1 atm.

A solute with a high solubility may be advantageous because if the composition of the invention is a solution, it may enable the solution to have a high concentration of solutes, which may in turn provide a high osmolarity or osmotic strength. Compositions with a high osmolarity or osmotic strength may assist with the antimicrobial efficacy of the composition because they may reduce the amount of water available for microbes or draw water away from microbes, and may assist in wound healing and wound debridement.

Fructose is a particularly preferred solute because it has a solubility of about 375g/100g water at 20°C and 1 atm. Consequently, the solute may be fructose.

According to the invention, the liquid or gel composition comprises: a purified enzyme that is able to convert a substrate to release hydrogen peroxide; a purified substrate for the enzyme; and a solute that is a sugar with a solubility of at least 100g/100g water at 20°C and 1 atm, and a polymer, wherein the composition has a water activity of less than 0.6 and substantially no hydrogen peroxide and wherein the composition does not comprise honey.

In other embodiments, the sugar may have a solubility of at least 200g/100g water at 20°C and 1 atm, or at least 300g/100g water at 20°C and 1 atm.

The composition may comprise: a purified enzyme that is able to convert a substrate to release hydrogen peroxide; a purified substrate for the enzyme; and purified fructose wherein the composition does not include sufficient free water to allow the enzyme to convert the substrate. The composition may comprise: purified glucose oxidase; purified glucose; and purified fructose, wherein the composition does not include sufficient free water to allow the enzyme to convert the substrate.

Compositions of the invention are in the form of a liquid or a gel. The liquids may be solutions.

Compositions of the invention which are liquids or solutions may comprise at least 70%, by weight of substrate and solute, more preferably at least 75%, by weight of substrate and solute, and even more preferably, at least 80% by weight of substrate and solute . For example, in a preferred embodiment, the composition comprises glucose and fructose. Preferably, the glucose and fructose is present in a total amount of at least 80%, by weight.

In some embodiments, the solute with a solubility of at least 100g/100g water at 20°C and 1 atm, at least 200g/100g water at 20°C and 1 atm or at least 300g/100g water at 20°C and 1 atm, may be the purified substrate. So, for example, a composition of the invention may comprise fructose and fructose oxidase.

Compositions of the invention may thus comprise only one sugar which is the solute and the substrate, and one enzyme for converting the substrate and generating hydrogen peroxide.

In some embodiments, the solute with the solubility of at least 100g/100g water at 20°C and 1 atm, at least 200g/100g water at 20°C and 1 atm or at least 300g/100g water at 20°C and 1 atm, may be distinct from the purified substrate. For example, a composition of the invention may comprise glucose, glucose oxidase and fructose.

The composition may be a liquid or solution comprising: a purified enzyme that is able to convert a substrate to release hydrogen peroxide; a purified substrate for the enzyme; and a solute that is a sugar with a solubility of at least 100g/100g water at 20°C and 1 atm, wherein the composition does not include sufficient free water to allow the enzyme to convert the substrate, and wherein the substrate and the solute provides at least 70% by weight, more preferably at least 75% by weight, or most preferably at least 80% by weight of the composition.

In other embodiments, the solute has a solubility of at least 200g/100g water at 20°C and 1 atm or more preferably at least 300g/100g water at 20°C and 1 atm.

The solute with a solubility of at least 100g/100g water at 20°C and 1 atm, 200g/100g water at 20°C and 1 atm, or 300g/100g water at 20°C and 1 atm (e.g. fructose), is preferably present in an amount of at least 40%, preferably at least 50%. The purified substrate (e.g. glucose) may be present in a composition of the invention in an amount of at least 20% by weight, preferably at least 25% by weight, more preferably at least 30% by weight. So, in one example, a solute (e.g. fructose) is present in an amount of 40 to 60% by weight and a substrate (e.g. glucose) is present in an amount of 20 to 40% by weight. In another example, a substrate (e.g. glucose) is present in an amount of 25 to 35% by weight and a solute (e.g. fructose) is present in an amount of 45 to 55%, by weight.

In preferred embodiments, the purified substrate is a sugar (e.g. glucose) and the solute is a sugar (e.g. fructose).

Compositions of the invention which are liquids or solutions may comprise at least 70%, by weight sugar, more preferably at least 75%, by weight sugar, and even more preferably, at least 80%, by weight, sugar. For example, in a preferred embodiment, the composition comprises glucose and fructose. Preferably, the glucose and fructose is present in an amount of at least 80% by weight of the composition.

The composition may be a liquid or solution comprising: a purified enzyme that is able to convert a substrate to release hydrogen peroxide; a purified substrate for the enzyme, wherein the composition does not include sufficient free water to allow the enzyme to convert the substrate, and wherein the composition comprises at least 70% by weight sugar, more preferably at least 75% by weight sugar, or most preferably at least 80% by weight sugar.

Preferably, the composition comprises at least two sugars (e.g. including glucose and fructose). The composition may comprise a maximum of two sugars (e.g. only glucose and fructose).

In compositions of the invention that are solutions or liquids, water may be present in an amount which is less than 20% by weight, but preferably greater than 10% by weight, more preferably greater than 15%, by weight. For example, water may be present in an amount between 10 and 20%, by weight, or in an amount of 10 to 20% by weight.

Compositions of the invention may comprise a buffer. An example of a suitable buffer is a citric acid/NaOH buffer, such as a 50 mMol citric acid/Na OH buffer. Compositions of the invention may be buffered at a pH of 5 or less, e.g. 3 to 5 (such as about pH 4). Alternatively, compositions of the invention may be buffered at a pH greater than 5, e.g. 6 to 8 (such as about pH 7).

Compositions of the invention may have a viscosity, such as a dynamic viscosity, of at least 5000 mPas at 20°C, more preferably at least 7500 at 20°C. Compositions of the invention may have a viscosity of 5000 to 20000 mPas at 20°C, more preferably 7500 to 12000 mPas at 20°C. A viscous solution or liquid may be afforded by high concentrations of sugars or sugar derivatives and may provide a similar viscosity to honey. A high viscosity may be beneficial in allowing a composition to remain in contact with a wound. Alternatively, a viscous solution or liquid may be afforded by the presence of, for example, non-aqueous solvents, polymers or hydrocolloid gelling agents as described herein.

Compositions of the invention may comprise at least 90% by dry weight of the substrate and the solute, combined. Compositions of the invention may comprise at least 95% by dry weight of the substrate and the solute, combined.

The composition may comprise: a purified enzyme that is able to convert a substrate to release hydrogen peroxide; a purified substrate for the enzyme and a solute wherein the composition does not include sufficient free water to allow the enzyme to convert the substrate and wherein the composition comprises at least 90%, by dry weight, of the substrate and the solute, combined.

Compositions of the invention may comprise at least 90% dry weight of sugar. Compositions of the invention may comprise at least 95% by dry weight of sugar.

The composition may comprise: a purified enzyme that is able to convert a substrate to release hydrogen peroxide; a purified substrate for the enzyme, wherein the composition does not include sufficient free water to allow the enzyme to convert the substrate and wherein the composition comprises at least 90% (preferably at least 95%), by dry weight, sugar.

Compositions of the invention may comprise at least 60%, dry weight of the solute. The substrate may be at least 30%, dry weight of the composition.

The composition may comprise: a purified enzyme that is able to convert a substrate to release hydrogen peroxide; a purified substrate for the enzyme, and a solute, wherein the composition does not include sufficient free water to allow the enzyme to convert the substrate, and wherein the composition comprises at least 60%, dry weight, of the solute and/or at least 30% dry weight of the substrate.

The following compositions comprising a precursor-substrate are not according to the invention unless they do comprise a purified substrate for the enzyme that is able to convert said substrate to release hydrogen peroxide.

For compositions which comprise a precursor-substrate, the composition preferably comprises one or more purified enzymes for converting the precursor-substrate to the substrate for the enzyme. However, in some embodiments, the precursor-substrate may not necessarily be converted to the substrate enzymatically. For example, for some precursor substrates, addition of water may be enough for conversion. Alternatively or additionally, compositions may comprise non-enzymatic catalysts.

So, compositions which comprise a precursor-substrate may comprise a first enzyme that is able to convert the substrate to release hydrogen peroxide, and a second enzyme that is able to convert the precursor-substrate to the substrate for the first enzyme.

The precursor-substrate is preferably a carbohydrate, such as a polysaccharide, or a sugar e.g. a disaccharide, or sugar derivative,

For example, the precursor-substrate may be sucrose, the first enzyme may be glucose oxidase and the second enzyme may be invertase.

In another example, the precursor-substrate may be maltose, the first enzyme may be glucose oxidase and the second enzyme may be maltase.

Compositions which comprise a precursor-substrate may comprise an enzyme (preferably a purified enzyme) that is able to convert the substrate to release hydrogen peroxide, and at least two enzymes (e.g. second and third enzymes, preferably purified enzymes) that are able to convert the precursor-substrate to the substrate for the first enzyme.

For example, the precursor-substrate may be starch, the first enzyme may be glucose oxidase and the second and third enzymes may be amylase and maltase.

For example, the precursor-substrate may be cellulose, the first enzyme may be glucose oxidase and the second and third enzymes may be cellulose and beta-glucosidase.

In some embodiments, compositions of the invention may comprise both a substrate that can be converted by the enzyme to generate hydrogen peroxide, and a precursor-substrate that can be converted to the substrate. The combined weight of the substrate and the precursor substrate may be at least 30% by dry weight of the composition. The combined dry weight of the substrate, precursor-substrate and the solute may be at least 90%, preferably at least 95% of the composition.

Compositions of the invention preferably contain substantially no zinc oxide. Compositions of the invention preferably contain essentially no zinc oxide.

Compositions of the invention preferably contain substantially catalase. Compositions of the invention preferably contain essentially catalase.

Compositions of the invention preferably contain substantially no peroxidase. Compositions of the invention preferably contain essentially no peroxidase.

Compositions of the invention may comprise thickening or gelling agents that increase the viscosity of a liquid without substantially changing its other properties. Suitable gelling or thickening agents include hydroxyethyl-cellulose or hydrocolloids.

Compositions of the invention preferably do not comprise an unrefined substance. The term "unrefined" is used herein to refer to substances that have not been processed into a pure form. Unrefined substances include substances that may have been concentrated, for example by drying or boiling.

Compositions of the invention preferably do not include one or more substrates from a natural source (termed herein a "natural substance"). Examples of natural substances include substances from a plant source, including from sap, roots, nectar, flowers, seeds, fruit, leaves, or shoots.

Preferably, compositions of the invention do not comprise an unrefined natural substance. Compositions of the invention do not comprise honey.

A composition of the invention may comprise at least one suitable antimicrobial or immunostimulatory component, excipient or adjuvant, or any other suitable component where it is desired to provide ability to generate antimicrobial activity. Preferably, however, the compositions do not include any antibiotic.

Compositions of the invention may include an antibiotic. A suitable antibiotic may be co-amoxiclav. Administering compositions of the invention with an antibiotic such as co-amoxiclav may provide a synergistic effect, such as in the treatment of biofilms, e.g. biofilms comprising NTHi.

According to the invention there may be provided a composition as defined herein for use in treating an infection (such as an infection comprising a biofilm), wherein the composition is administered with an antibiotic. The infection may comprise *Haemophilus influenza* (e.g. non-typeable *haemophilus influenza*; NTHi). The antibiotic may be co-amoxiclav, although other antibiotics could be used. The administration may be Combined, Concurrent, or Sequential.

Described - but not according to the claimed invention - is a kit comprising a composition of the invention and, separately, an antibiotic (e.g. co-amoxiclav).

Compositions of the invention comprise a polymer. The polymer in the composition may be any medically acceptable polymer, such as any Food and Drug Administration-approved (FDA-approved) polymer.

In some embodiments, the polymer may be a synthetic polymer. In some embodiments, the polymer is a natural polymer.

Optionally, the polymer is water soluble. The polymer may be soluble in an organic, or non-aqueous, solvent. The polymer may be soluble in a mixture of an aqueous and non-aqueous solvent. The polymer may be biodegradable or bioerodable. The polymer may be a co-polymer. In some embodiments, the polymer is selected from polyethylene oxide (or polyethylene glycol), polyvinyl alcohol and polyvinylpyrrolidone.

Other polymers may include poly(lactic-co-glycolic acid), polyglycolic acid, polylactic acid, polycaprolactone or polymeric surfactants. Another suitable polymer may be phosphino-carboxylic acid (PCA).

Further polymers may include polysaccharides such as cellulose (which includes derivatives such as hydroxypropyl methyl cellulose and hydroxypropyl cellulose), alginate, gelatin or cyclodextrins. Suitable polymers may also include chitosan or hyaluronic acid.

Compositions of the invention may comprise up to 50%, 25%, 10% or 5% by weight of the polymer. For example, the composition may comprise from 0.5 to 3% by weight of the polymer. Optionally, the polymer may be from 0.5 to 50% by weight of the composition.

Compositions of the invention may be electrospinnable.

There is also provided a method of producing a fiber, comprising electrospinning an electrospinnable composition of the invention. Such method however does not form part of the claimed invention.

A fiber, preferably a nanofiber, may comprise: a purified enzyme that is able to convert a substrate to release hydrogen peroxide; and a purified substrate for the enzyme, and optionally a purified precursor substrate that can be converted to a substrate for the enzyme. The fiber preferably does not comprise sufficient free water to allow the enzyme to convert the substrate. The fiber may comprise one or more solutes as described herein. Such fiber however does not form part of the claimed invention.

A wound dressing or nanofibrous mat may comprise one or more fibers of the disclosure.

The electrospinnable composition may be a solution. The solvent in the solution may be, or may comprise, water. The solvent may comprise an aqueous and/or non-aqueous solvent, such as an organic solvent.

The electrospinnable composition may comprise one or more electrospinnable components. Any electrospinnable component which facilitates formation of a fiber or dressing according to the invention, may be suitable. Preferably, the one or more electrospinnable component is an electrospinnable polymer. In some embodiments, the polymer may be a synthetic polymer. In some embodiments, the polymer is a natural polymer. In some embodiments, the electrospinnable polymer is selected from polyethylene oxide, polyvinyl alcohol and polyvinylpyrrolidone. Other polymers may include polycaprolactone or phosphino-carboxylic acid (PCA).

The electrospinnable component is preferably biocompatible. Optionally, the electrospinnable component is water soluble. The electrospinnable component may be soluble in an organic, or non-aqueous, solvent. The electrospinnable component may be soluble in a mixture of an aqueous and non-aqueous solvent. Suitable non-aqueous solvents may be, or may comprise, glycerol, dimethyl sulphoxide, ethylene glycol or propylene glycol.

The electrospinnable composition may comprise up to 50%, 25%, 10% or 5% by weight of the electrospinnable component. Optionally, the electrospinnable component may be from 1 to 50% by weight of the composition. The electrospinnable composition may comprise up to 30%, 20% or 10% by weight of the unrefined natural substance.

It will be appreciated that the relative amounts of the electrospinnable component and the solvent may be varied to alter the properties of the fibers.

A fiber that has been formed from the electrospinnable composition may comprise up to 80% by weight of the unrefined natural substance. The fiber may comprise 20% or more, by weight, of the electrospinnable component.

Compositions of the invention may comprise salt. Alternatively, the salt may be provided in a kit separately from the rest of the composition. Accordingly, there is also provided according to the invention a kit comprising: a composition for generating anti-microbial activity that comprises an enzyme that is able to convert a substrate to release hydrogen peroxide, and a substance that includes a substrate for the enzyme; and, separately, a salt. The kit may further include instructions, for example, for mixing of the components of the kit, and their use to treat a microbial infection.

The salt may be provided in dry form, or in aqueous solution. The salt may comprise sodium chloride.

The composition in dry form may comprise a ratio of the composition for generating anti-microbial activity to the salt, for example, of from 1:2 to 5:1, or from 2:3 to 3:2.

The composition may comprise, for example, 1-99%, 1-80%, 1-70%, 1-60%, 1-50%, 1-40%, 1-30%, 1-20%, or 1-10%, by weight, of the composition for generating anti-microbial activity.

The composition may comprise, for example, 1-99%, 1-80%, 1-70%, 1-60%, 1-50%, 1-40%, 1-30%, 1-20%, or 1-10%, by weight, of the salt.

The composition may be provided as an aqueous mixture. The aqueous mixture may be an isotonic or hypertonic mixture. The aqueous mixture may comprise, for example, 0.1-20% w/v salt, suitably 0.25-10%, 0.25-10%, 0.25-5%, 0.25-3%, 0.5-10%, 0.5-5%, or 0.5-3%, for example 0.9% w/v salt. The aqueous mixture may comprise, for example, 1-300%, 1-250%, 1-200%, 1-150%, 1-100%, 1-50%, 1-40%, 1-30%, 1-20%, or 1-10% w/v of the composition for generating anti-microbial activity. The aqueous mixture may comprise, for example, 10-300%, 10-250%, 10-200%, 10-150%, 10-100%, 10-50%, 10-40%, 10-30%, or 10-20% w/v of the composition for generating anti-microbial activity. The aqueous mixture may comprise, for example, 50-300%, 50-250%, 50-200%, 50-150%, or 50-100% w/v of the composition for generating anti-microbial activity. The aqueous mixture may comprise, for example, 0.1-20%, 0.1-10%, 0.1-5%, 0.1-1% w/v of sodium bicarbonate. Aqueous compositions as such are not within the scope of the present claims unless the water activity is less than 0.6.

Compositions of the invention, for example those comprising salt, may be used as a nasal douche, for example to prevent or treat nasal microbial infection, sinusitis, rhinitis, CRS, nasal allergy, cold or flu symptoms, congestion, or dryness. The compositions of the invention may be used to prevent or treat a microbial infection, for example a microbial infection that comprises a biofilm, or a microbe that is capable of forming a biofilm. The microbial infection that comprises a biofilm may be a nasal microbial infection, or the microbe that is capable of forming a biofilm may be part of a nasal microbial infection.

To use a composition or mixture of the invention as a nasal douche, it may be poured into one nostril and allowed to run out through the other, while the mouth is kept open to breathe, using gravity as an aid. Alternatively, some form of positive pressure may be applied to facilitate rinsing. For example, bottles made of flexible plastic, optionally with special tips to fit the nostril, can be squeezed to exert positive pressure of the mixture flowing through the sinuses while the mouth is kept open at all times in order to breathe and prevent snorting the liquid down the throat. Irrigation machines that utilize electric motor-driven pumps are also available. Some nasal irrigation systems that apply pressure have an anti-backwash valve to prevent used saltwater solution from flowing back into the nasal cavity. Such uses and/or irrigation machines are not part of the claimed invention.

Compositions or mixtures of the invention may be provided in a neti pot, a container used to administer nasal douche. Neti pots are typically made of metal, glass, ceramic or plastic. They rely on gravity, along with head positioning and repeated practice in order to rinse the outer sinus cavities. Typically they have a spout attached near the bottom, sometimes with a handle on the opposite side.

Compositions of the invention may comprise a non-aqueous solvent. In compositions of the invention that comprise a non-aqueous solvent, the non-aqueous solvent may comprise ethanol, dimethyl sulphoxide, glycerol, ethylene glycol or propylene glycol. Preferably, the non-aqueous solvent is or comprises glycerol. Glycerol may act as a humectant and so compositions comprising glycerol may assist in softening or moisturising dry skin.

Solubility parameters and viscosity parameters for various non-aqueous solvents are shown in the following table.

| **Substance** | **Hansen solubility parameter** | | | | **Solubility of Glucose g/100ml of solvent** |
|---|---|---|---|---|---|
| | **δₜ/MPa^{1/2}** | **δ_{d}/MPa^{1/2}** | **δₚ/MPa^{1/2}** | **δₕ/MPa^{1/2}** | |
| Ethanol | 26.5 | 15.8 | 8.8 | 19.4 | 1.94 g |
| Dimethyl sulphoxide | 26.7 | 18.1 | 16.4 | 10.2 | 54 g |
| Propylene glycol | 30.2 | 16.8 | 9.4 | 23.3 | |
| Ethylene glycol | 32.9 | 17.0 | 11.0 | 26 | |
| Glycerol | 36.1 | 17.4 | 12.1 | 29.3 | |
| Water | 47.8 | 15.6 | 16.0 | 42.3 | 90g |

| **Substance** | **Viscosity** |
|---|---|
| Ethanol | 1.04mPa.s |
| Dimethyl sulphoxide | 1.99mPa.s |
| Glycerol | 1412mPa.s |
| Ethylene glycol | 16.1mPa.s |
| Propylene glycol | 42mPa.s |
| Water | 1.3mPa.s |

In preferred embodiments, non-aqueous solvents may be selected so that they have solubility parameters in the range of the non-aqueous solvents exemplified in the tables below. For example, δₜ/MPa^{1/2} may be from 26 to 50, such as 26.5 to 47.8. δ_{d}/MPa^{1/2} may be from 15 to 19, such as 15.6 to 18.1. δₚ/MPa^{1/2} may be from 8 to 16, such as 8.8 to 16. δₕ/MPa^{1/2} may be from 10 to 45, such as 10.2 to 42.3.

The non-aqueous solvent may be selected depending on the desired viscosity. For example, if a greater viscosity is desired, glycerol may be preferred.

In preferred embodiments of compositions comprising a non-aqueous solvent, the compositions may comprise at least 10% by weight of the non-aqueous solvent. In other embodiments, the composition may comprise at least 20% by weight of the non-aqueous solvent. In other embodiments, the composition may comprise at least 25% by weight of the non-aqueous solvent. In other embodiments, the composition may comprise at least 50% by weight of the non-aqueous solvent. In some embodiments, the composition may comprise at least 75% by weight of the non-aqueous solvent. The amount of aqueous solvent may be varied depending on the intended application of the composition. For example, sprayable compositions, or compositions for use with an antibacterial wipe may comprise higher levels of the non-aqueous solvent, such that the compositions have a lower viscosity. In some embodiments, the amount of non-aqueous solvent in the composition may be 50-90%, by weight.

For some applications, it may be desirable to have compositions that comprise lower amounts of non-aqueous solvent, such as compositions for use in forming wound dressings. Consequently, some compositions may comprise a maximum amount of non-aqueous solvent. The maximum amount of non-aqueous solvent in the composition may be 50% by weight or less. In some embodiments, the amount of non-aqueous solvent in the composition may be 1-50, 5-50 or 10-50%, by weight.

If compositions of the invention are to be used to coat a substrate, such as a fabric, the weight of the composition is preferably at least 100g per square metre of the substrate. In other embodiments, the weight of the composition may be at least 200g per square metre of the substrate. In other embodiments, the weight of the composition may be at least 300g per square metre of the substrate.

Compositions of the invention may comprise an amount of water that may typically be expected to permit the enzyme to convert the substrate, or the precursor-substrate to be converted to the substrate. For example, compositions of the invention may comprise greater than 20% by weight of water, or greater than 30% by weight of water. In compositions according to the invention the water activity has however to be less than 0.6. This water may not be available, or free, to allow the enzyme to convert the substrate because the non-aqueous solvent may bind or lock in the water. A non-aqueous solvent may thus act as a humectant. The non-aqueous solvent or humectant may reduce the water activity (a_{w}) of the composition.

Compositions of the invention may thus comprise a humectant. In some embodiments, the humectant is not a non-aqueous solvent. However, in preferred embodiments, the humectant is a non-aqueous solvent.

Viscous compositions may limit its range of applications. Inclusion of both a non-aqueous solvent or humectant, in the composition may reduce the viscosity of the composition, and less viscous compositions may be beneficial if, for example, the composition is to be readily sprayable. A suitable viscosity for a composition of the invention may be 100 mPa.s. or less at 20°C. In some embodiments, a suitable viscosity may be 75 mPa.s. or less at 20°C. In some embodiments, a suitable viscosity may be 50 mPa.s. or less at 20°C.

The relative amounts of the humectant or non-aqueous solvent, and the additional water, in a composition of the invention, may be selected such that the composition does not comprise sufficient free water to allow the enzyme to convert the substrate. When such a composition is contacted with even more water, for example if the composition is diluted or if the composition comes into contact with fluid from a wound, there may be sufficient free water for the enzyme to convert the substrate and produce hydrogen peroxide.

In compositions of the invention, the water activity (a_{w}) is less than 0.6, preferably less than 0.5.

In compositions of the invention comprising humectant or non-aqueous solvent, the amount of humectant or non-aqueous solvent may be at least 30% by weight. In other embodiments, the amount of humectant or non-aqueous solvent may be at least 40% by weight. In some embodiments, the amount of humectant or non-aqueous solvent may be at least 50% by weight. The amount of humectant or non-aqueous solvent may be 75% or less, by weight. The amount of humectant or non-aqueous solvent may be 60% or less by weight. In some embodiments, the amount of humectant or non-aqueous solvent may be 30-75% or 40-60% by weight.

Compositions of the invention may comprise a haemostatic agent or blood clotting agent. For example, compositions of the invention may comprise one or more blood coagulation factors, such as fibrinogen or thrombin. Compositions of the invention may comprise other naturally-occurring haemostats such as chitin.

Alternatively, or additionally, compositions of the invention may comprise a synthetic haemostatic agent, such as an agent comprising carriers to each of which a plurality of fibrinogen binding peptides are immobilised. Such agents may include those described in WO 2008/065388 and WO 2015/104544, which are able to form a biogel on contact with fibrinogen, and in the absence of thrombin.

Compositions of the invention may comprise a lipophilic phase and an aqueous phase.

A composition of the invention may be in the form of a colloid or a suspension.

The term "colloid" is used herein to refer to a homogeneous non-crystalline substance consisting of large molecules or ultramicroscopic particles of one substance dispersed through a second substance. Colloids include gels, sols, and emulsions. The particles do not settle, and cannot be separated out by ordinary filtering or centrifuging like those in a suspension.

The term "suspension" is used herein to refer to a mixture in which small particles of a substance are dispersed throughout a liquid. If a suspension is left undisturbed, the particles are likely to settle to the bottom. The particles in a suspension are larger than those in either a colloid or a solution.

A composition of the invention may be in the form of an emulsion. The term "emulsion" is used herein to refer to a fine dispersion of minute droplets of one liquid in another in which it is not soluble or miscible. An emulsion of the invention may be an oil and water emulsion, in particular an oil-in-water emulsion, or a water-in-oil emulsion. The composition may be a micro-emulsion.

The composition may comprise a first phase (or first liquid, or first component) and a second phase (or second liquid, or second component), a purified enzyme that is able to convert a substrate to release hydrogen peroxide; and a purified substrate for the enzyme.

The first phase and the second may be immiscible. For example, the first phase may be less polar than the second phase. The first phase may be a non-polar phase such as a lipophilic phase or a hydrophobic phase e.g. an oil. The second phase may be a polar phase, such as an aqueous phase. The second phase may comprise a non-aqueous solvent. Droplets or micelles of the second phase may be dispersed within the first phase.

Preferably, compositions comprising a first phase and a second phase do not comprise sufficient free water to allow the enzyme to convert the substrate.

The composition may comprise a lipophilic phase; an aqueous phase; a purified enzyme that is able to convert a substrate to release hydrogen peroxide; and a purified substrate for the enzyme.

The composition may comprise: an oil; an emulsifier; an enzyme that is able to convert a substrate to release hydrogen peroxide; and a purified substrate for the enzyme.

The second phase may comprise water and/or non-aqueous solvent. The enzyme and the substance of the composition may be dissolved in the water and/or non-aqueous solvent.

It is conceivable that, in some embodiments, the second phase may not comprise water or may comprise substantially no water. In such circumstances, the second phase may be described as non-aqueous. For example, the enzyme and the substance comprising a substrate for the enzyme may be dissolved in a non-aqueous solvent. The non-aqueous solvent may be immiscible with respect to the first phase e.g. lipophilic phase.

In some embodiments, the enzyme that is able to convert a substrate to release hydrogen peroxide and the substance that includes a substrate for the enzyme may be contained within micelles dispersed within the first phase, e.g. lipophilic phase.

In some compositions, the composition may be in the form of a double emulsion. For example, droplets containing the enzyme that is able to convert a substrate to release hydrogen peroxide and the substance that includes a substrate for the enzyme may be dispersed within globules of a lipophilic phase (e.g. oil globules) and globules may be dispersed within an aqueous phase. Such a double emulsion may be termed a water-in-oil-in-water type (W/O/W) emulsion.

A composition of the invention may further comprise an emulsifying agent (or emulsifier). Emulsions can be stabilized by adsorption of surface active agents (emulsifying agents) at the emulsion interface. Emulsifying agents lower the interfacial tension to maintain the droplets in a dispersed state. An emulsifying agent has a hydrophilic part and a lipophilic part. It is possible to calculate the relative quantities of an emulsifying agent(s) necessary to produce the most physically stable emulsions for a particular formulation with water combination. This approach is called the hydrophilic-lipophilic balance (HLB) method ("The HLB SYSTEM a time-saving guide to emulsifier selection" ICI Americas Inc., Wlimington, Delaware 19897, 1976, revised 1980). Each emulsifying agent is allocated an HLB number representing the relative properties of the lipophilic and hydrophilic parts of the molecule. High numbers (up to a theoretical number of 20), indicates an emulsifying agent exhibiting mainly hydrophilic or polar properties, whereas low numbers represent lipophilic or non-polar characteristics. According to the HLB System, all fats and oils have a Required HLB. Emulsions with optimal performance can be yielded by matching the HLB requirement with the emulsifying agent's HLB value. For an oil-in-water emulsion, the more polar the oil phase the more polar the emulsifying agent(s) must be. For example, to emulsify Soybean Oil, which has a Required HLB of 7, according to the HLB system, it would be necessary to use an emulsifying agent, or blend of emulsifying agents, with an HLB of 7 ± 1. The HLB of emulsifying agents can be calculated or determined through trial and error.

Thus, the lipophilic phase of a composition of the invention may require an emulsifying agent of a particular HLB number in order to ensure a stable product. The lipophilic phase of a composition of the invention may comprise an oil or a wax. Examples of oils and waxes (by their International Nomenclature of Cosmetic Ingredients, INCI, name) for use in a lipophilic phase of a composition of the invention (with their respective Required HLBs) include the following:

| | |
|---|---|
| Aleurites Moluccana Seed Oil [7] | Grape (Vitis Vinifera) Seed Oil [7] |
| Almond Oil NF [6] | Hybrid Safflower (Carthamus Tinctorius) Oil [9] |
| Anhydrous Lanolin USP [10] | Isopropyl Myristate [11.5] |
| Apricot Kernel Oil [7] | Isopropyl Palmitate [11.5] |
| Avocado (Persea Gratissima) Oil [7] | Jojoba (Buxus Chinensis) Oil [6.5] |
| Babassu Oil [8] | Lanolin [10] |
| Beeswax [12] | Macadamia (Ternifolia) Nut Oil [7] |
| Borage (Borago Officinalis) Seed Oil [7] | Mangifera Indica (Mango) Seed Butter [8] |
| Brazil Nut Oil [8] | Mineral Oil [10.5] |
| C12-15 Alkyl Benzoate [13] | Myristyl Myristate [8.5] |
| Cannabis Sativa Seed Oil [7] | Olive (Olea Europaea) Oil [7] |
| Canola Oil [7] | Oryza Sativa (Rice Bran) Oil [7] |
| Caprylic/Capric Triglyceride [5] | Peanut Oil NF [6] |
| Carrot (Daucus Carota Sativa) Seed Oil [6] | Petrolatum [7] |
| Castor (Ricinus Communis) Oil [14] | PPG-15 Stearyl Ether [7] |
| Ceresin [8] | Retinyl Palmitate [6] |
| Cetearyl Alcohol [15.5] | Safflower (Carthamus Tinctorius) Oil [8] |
| Cetyl Alcohol [15.5] | Sesame (Sesamum Indicum) Oil [7] |
| Cetyl Esters [10] | Shea Butter (Butyrospermum Parkii) [8] |
| Cetyl Palmitate [10] | Soybean (Glycine Soja) Oil [7] |
| Coconut Oil [8] | Stearic Acid [15] |
| Daucus Carota Sativa (Carrot) Root Extract [6] | Stearyl Alcohol [15.5] |
| Diisopropyl Adipate [9] | Sunflower (Helianthus Annus) Oil [7] |
| Dimethicone [5] | Sweet Almond (Prunus Amygdalus Dulcis) Oil [7] |
| Dog Rose (Rosa Canina) Hips Oil [7] | Theobroma Cacao (Cocoa) Seed Butter [6] |
| Emu Oil [8] | Tocopherol [6] |
| Evening Primrose Oil [7] | |

In some embodiments, the lipophilic phase of a composition of the invention comprises a beeswax.

In some embodiments, the lipophilic phase is an oil. In some embodiments, the oil is selected from olive oil, corn oil, vegetable oil, sunflower oil or paraffin oil. In a preferred embodiment, the oil may be olive oil. In another preferred embodiment, the oil may be paraffin oil.

Water-in-oil emulsifying agents for use in compositions of the invention may have an HLB value in the range 3-6. Oil-in-water emulsifying agents for use in compositions of the invention may have an HLB value in the range 8-18. Examples of emulsifying agents (by their INCI name) for use in compositions of the invention (with their HLB numbers) include the following:

| | |
|---|---|
| Calcium Stearoyl Lactylate [HLB = 5.1 ± 1] | Oleth-20 [HLB = 15.3 ± 1] |
| Ceteareth-20 [HLB = 15.2 ± 1] | PEG-100 Stearate [HLB = 18.8 ± 1] |
| Cetearyl Glucoside [HLB = 11 ± 1] | PEG-20 Almond Glycerides [HLB = 10 ± 1] |
| Ceteth-10 [HLB = 12.9 ± 1] | PEG-20 Methyl Glucose Sesquistearate [HLB = 15 ± 1] |
| Ceteth-2 [HLB = 5.3 ± 1] | PEG-25 Hydrogenated Castor Oil [HLB = 10.8 ±1] |
| Ceteth-20 [HLB = 15.7 ± 1] | PEG-30 Dipolyhydroxystearate [HLB = 5.5 ± 1] |
| Cocamide MEA [HLB = 13.5 ± 1] | PEG-4 Dilaurate [HLB = 6 ± 1] |
| Glyceryl Laurate [HLB = 5.2 ± 1] | PEG-40 Sorbitan Peroleate [HLB = 9 ± 1] |
| Glyceryl Stearate [HLB = 3.8 ± 1] | PEG-60 Almond Glycerides [HLB = 15 ± 1] |
| Glyceryl Stearate (and) PEG-100 Stearate [HLB = 11 ±1] | PEG-8 Laurate [HLB = 13 ± 1] |
| Glyceryl Stearate SE [HLB = 5.8 ± 1] | PEG-80 Sorbitan Laurate [HLB = 19.1 ± 1] |
| Glycol Distearate [HLB = 1 ± 1] | Polysorbate 20 [HLB = 16.7 ± 1] |
| Glycol Stearate [HLB = 2.9 ± 1] | Polysorbate 60 [HLB = 14.9 ± 1] |
| Isoceteth-20 [HLB = 15.7 ± 1] | Polysorbate 80 [HLB = 15 ± 1] |
| Isosteareth-20 [HLB = 15 ± 1] | Polysorbate 85 [HLB = 11 ± 1] |
| Lauramide DEA [HLB = 15 ± 1] | Sodium Stearoyl Lactylate [HLB = 8.3 ± 1] |
| Laureth-23 [HLB = 16.9 ± 1] | Sorbitan Isostearate [HLB = 4.7 ± 1] |
| Laureth-4 [HLB = 9.7 ± 1] | Sorbitan Laurate [HLB = 8.6 ± 1] |
| Lecithin [HLB = 4 ± 1] | Sorbitan Oleate [HLB = 4.3 ± 1] |
| Lecithin [HLB = 9.7 ± 1] | Sorbitan Sesquioleate [HLB = 3.7 ± 1] |
| Linoleamide DEA [HLB = 10 ± 1] | Sorbitan Stearate [HLB = 4.7 ± 1] |
| Methyl Glucose Sesquistearate [HLB = 6.6 ±1] | Sorbitan Stearate (and) Sucrose Cocoate [HLB = 6 ± 1] |
| Oleth-10 [HLB = 12.4 ± 1] | Sorbitan Trioleate [HLB = 1.8 ± 1] |
| Oleth-10 / Polyoxyl 10 Oleyl Ether NF [HLB = 12.4 ±1] | Stearamide MEA [HLB = 11 ± 1] |
| Oleth-2 [HLB = 4.9 ± 1] | Steareth-2 [HLB = 4.9 ± 1] |
| Oleth-20 [HLB = 12.4 ± 1] | Steareth-21 [HLB = 15.5 ± 1] |

In some embodiments, an emulsifying agent of a composition of the invention comprises a lecithin.

Emulsifying agents include ionic or non-ionic surfactants, and lipophilic fatty amphiles (for example, fatty alcohols or fatty acids). Non-ionic surfactants may be preferred since they may be less irritating to skin that anionic or cationic surfactants.

Other examples of suitable emulsifying agents include: Surfactants: Sodium lauryl sulphate, Cetrimide, Cetomacrogol 1000, PEG 1000 monostearate, Triethanolamine stearate, Sodium stearate; Fatty amphiphiles: Cetostearyl alcohol, Cetyl alcohol, Stearyl alcohol, Glyceryl monostearate, Stearic acid, Phosphatidylcholine.

Examples of commercial emulsifying waxes include: Emulsifying wax BP (Cetostearyl alcohol, sodium lauryl sulphate), Emulsifying wax USNF (Cetyl alcohol, polysorbate), Cationic emulsifying wax BPC (Cetostearyl alcohol, cetrimide), Glyceryl monostearate S.E. (Glyceryl monostearate, sodium stearate), Cetomacrogol emulsifying wax BPC (Cetostearyl alcohol, cetomacrogol 1000), Polawax (Cetyl alcohol, non-ionic surfactant), Lecithin (Phosphatidylcholine, phosphatidylethanolamine, phosphatidylinositol, phosphatidic acid).

Surfactants for use in compositions of the invention may include one or more of TWEEN (e.g. TWEEN 80), SPAN (e.g. SPAN 80), Poloxamer (e.g. Poloxamer 407) and Polyglycerol polyricinoleate (PGPR). A preferred surfactant may be Poloxamer, such as Poloxamer 407. Another preferred surfactant may be PGPR.

Surfactants may include a surfactant polymer, or co-polymer. For example, a suitable surfactant may be a triblock copolymer consisting of a central hydrophobic block flanked by two hydrophilic blocks.

Compositions of the invention may comprise non-aqueous solvent. The non-aqueous solvent may be a polar solvent, such as a solvent with a dielectric constant of greater than 15. The non-aqueous solvent may be an organic solvent. For example, the solvent may be, or may comprise, glycerol, dimethylsulphoxide, propylene glycol or polyethylene glycol. The non-aqueous solvent may be immiscible with respect to the first phase e.g. the lipophilic phase (such as oil).

In a preferred embodiment, the non-aqueous solvent may be, or comprise, glycerol.

In some embodiments, compositions of the invention may comprise micelles, preferably reverse micelles. Within each micelle may be the enzyme and the substance (which may comprise an unrefined natural substance, such as honey), and outside of the micelle may be the first phase, e.g. the lipophilic phase (such as oil). Within each reverse micelle there may also be water and/or non-aqueous solvent. Within each micelle there may not be sufficient water for the enzyme to convert the substrate.

Compositions of the invention may comprise further components which may assist in reducing coalescence. Coalescence describes the situation in which two or more droplets, or micelles, combine to form a single droplet, or micelle. In order to reduce or prevent, coalescence, the strength of the interfacial film, *i.e.* the interface between the lipophilic phase and the aqueous phase, may be strengthened. This may be achieved, for example, by increasing the surfactant concentration, including an amphiphilic polymer, and/or by adding an alcohol, such as an aliphatic alcohol with 5-7 carbon atoms.

Compositions of the invention for topical application may be in the form, for example, of a cream, a lotion, or a lip balm, provided that the compositions are liquid or gel compositions.

The term "cream" is used herein to refer to a semi-solid emulsion of oil-in-water, or water-in-oil, for topical use. Oil-in-water (o/w) creams are composed of small droplets of oil dispersed in a continuous aqueous phase, and water-in-oil (w/o) creams are composed of small droplets of water dispersed in a continuous oily phase. Oil-in-water creams are less greasy and more easily washed off using water. Water-in-oil creams are more moisturising as they provide an oily barrier which reduces water loss from the outermost layer of the skin.

The term "cream" may also refer to a semi-solid emulsion in which droplets of a first phase are dispersed in a continuous second phase, or in which droplets of a second phase are dispersed in a continuous first phase. For example, the first phase may be less polar than the second phase. The first phase may be a non-polar phase such as a lipophilic phase or a hydrophobic phase e.g. an oil. The second phase may be a polar phase, such as an aqueous phase. The second phase may comprise a non-aqueous solvent. The second phase may comprise water and/or non-aqueous solvent. It is conceivable that, in some embodiments, the second phase may not comprise water or may comprise substantially no water. In such circumstances, the second phase may be described as non-aqueous. The non-aqueous solvent may be immiscible with respect to the first phase e.g. lipophilic phase.

The term "lotion" is used herein to refer to a liquid suspension or emulsion for topical application. A lotion may comprise finely powdered, insoluble solids held in suspension by suspending agents and/or surface-active agents, or an emulsion (particularly, an oil-in-water emulsion) stabilized by one or more surface-active agents. A lotion has lower viscosity than a cream.

The term "lip balm" is used herein the refer to a wax-like substance applied topically to the lips of the mouth to moisturize and relieve chapped or dry lips. Lip balm may include, for example, beeswax or carnauba wax, camphor, cetyl alcohol, lanolin, paraffin, and petrolatum, among other ingredients.

The ratio of the lipophilic phase to the aqueous phase, or the ratio of the first phase to the second phase, in a composition of the invention may be from 9:1 to 1:9, 8:1 to 1:8, 7:1 to 1:7, 6:1 to 1:6, 5:1 to 1:5, 4:1 to 1:4, 3:1 to 1:3, or 2:1 to 1:2 (v/v), for example from 4:1 to 1:4.

A composition of the invention may comprise 5-95%, 10-95%, 15-95%, 20-95%, 25-95%, 30-95%, 35-95%, 40-95%, 45-95%, 50-95%, 55-95%, 60-95%, 65-95%, 70-95%, 75-95%, 80-95%, 85-95%, or 90-95% (v/v) lipophilic phase, or first phase (including any emulsifying agent present).

Alternatively, a composition of the invention may comprise 5-95%, 5-90%, 5-85%, 5-80%, 5-75%, 5-70%, 5-65%, 5-60%, 5-55%, 5-50%, 5-45%, 5-40%, 5-35%, 5-30%, 5-25%, 5-20%, 5-15%, or 5-10% (v/v) lipophilic phase, or first phase (including any emulsifying agent present).

A composition of the invention may comprise 5-95%, 10-95%, 15-95%, 20-95%, 25-95%, 30-95%, 35-95%, 40-95%, 45-95%, 50-95%, 55-95%, 60-95%, 65-95%, 70-95%, 75-95%, 80-95%, 85-95%, or 90-95% (v/v) aqueous phase, or second phase.

Alternatively, a composition of the invention may comprise 5-95%, 5-90%, 5-85%, 5-80%, 5-75%, 5-70%, 5-65%, 5-60%, 5-55%, 5-50%, 5-45%, 5-40%, 5-35%, 5-30%, 5-25%, 5-20%, 5-15%, or 5-10% (v/v) aqueous phase, or second phase.

A composition of the invention may comprise 1-1500 units, 15-1500 units, 30-1500 units, 50-1500 units, 100-1500 units, 1-<685 units, 15-<685 units, 30-<685 units, 50-<685 units, 100-<685 units, 500-1000 units, 685-1000 units, or 100-500 units, of the enzyme, preferably glucose oxidase, per gram of the composition.

A composition of the invention may comprise no more than 85% water, for example no more than 80%, 70%, 60%, 50%, 40%, 30%, or 20% water, or less than 20% water, for example 10-19% water. A composition of the invention may comprise less than 20% (w/w). A composition of the invention may comprise less than 15% (w/w) water. A composition of the invention may comprise less than 12% (w/w) water. In each and any case the water activity of the composition according to the invention has to be less than 0.6.

A composition of the invention may comprise 10-60% (w/w) of non-aqueous solvent. In some embodiments, a composition of the invention may comprise 20-50% (w/w) of a non-aqueous solvent. In some embodiments, a composition of the invention may comprise 35-40% (w/w) of a non-aqueous solvent.

A composition of the invention may comprise 10-40 % (w/w) of the first phase, e.g. lipophilic phase (such as oil). The composition may comprise 20-30% (w/w) of the first phase, e.g. lipophilic phase (such as oil).

A composition of the invention may comprise 1-10% (w/w) emulsifier. The composition may comprise 1-5% (w/w) emulsifier. The emulsifier is preferably a surfactant.

A composition of the invention may comprise 20-50% (w/w) of non-aqueous solvent, 20-30% (w/w) of the first phase e.g. lipophilic phase (such as oil), 1-5% (w/w) emulsifier and 20-40% (w/w) of the substance which comprises a substrate for the enzyme.

A composition of the invention may comprise 10-60% (w/w) of non-aqueous solvent, 10-40% (w/w) of the first phase e.g. lipophilic phase (such as oil), 1-10% (w/w) emulsifier and 10-50% (w/w) of the substance which comprises a substrate for the enzyme.

A composition of the invention may comprise 35-45% (w/w) of non-aqueous solvent, 20-30% (w/w) of the first phase e.g. lipophilic phase (such as oil), 1-5% (w/w) emulsifier and 25-35% (w/w) of the substance which comprises a substrate for the enzyme.

A composition of the invention may comprise 30-60% (v/v) solvent, such as a non-aqueous, polar solvent.

A composition of the invention may comprise 30-60% (v/v) first phase, such as a lipophilic phase (e.g. oil),
A composition of the invention may comprise 1-10% (v/v) emulsifier such e.g. surfactant.

The ratio of the first phase to the second phase in a composition of the invention may be ≤1:1 (v/v), for example 0.1-1:1 (v/v). In some embodiments, the ratio of the first phase to the second phase is <0.6:1 (v/v), for example 0.1-<0.6:1 (v/v). In some embodiments, the ratio of the first phase to the second phase is ≤0.4:1 (v/v), for example 0.1-0.4:1 (v/v).

The first phase in a composition of the invention may be present at less than 60% (v/v) of the composition. In some embodiments, the first phase is present at 10% to less than 60% (v/v) of the composition. In some embodiments, the first phase is present at 10% to less than 50% (v/v) of the composition. In some embodiments, the first phase is present at 10% to less than 40% (v/v) of the composition. In some embodiments, the first phase is present at 10% to less than 30% (v/v) of the composition. In some embodiments, the first phase is present at 10% to less than 25% (v/v) of the composition.

A composition of the invention may comprise an emulsifier. In some embodiments, the emulsifier is present at up to 25% (v/v) of the composition, for example 1-25% (v/v) of the composition, 5-25% (v/v) of the composition, or 10-25% (v/v) of the composition.

A composition of the invention may be an emulsion. In particular embodiments, a composition of the invention is an emulsion that comprises reverse micelles. The reverse micelles may be formed by the second phase.

In some embodiments of a composition of the invention, the enzyme and the substrate is dissolved in the second phase.

In particular embodiments of the invention, the first phase is, or comprises paraffin oil.

In particular embodiments of the invention, the second phase is, or comprises glycerol.

In particular embodiments of the invention, the emulsifier is, or comprises Polyglycerol polyricinoleate (PGPR).

In some embodiments, a composition of the invention is a cream. Typically, the viscosity of an emulsion used as a cream will be higher than that of an emulsion used as a spray. A cream may be formed by including a viscosity-increasing agent, such as a thickener or gelling agent (for example a hydrocolloid) in the composition.

Hydrocolloids are a heterogeneous group of hydrophilic, long-chain polymers (polysaccharides or proteins) characterised by their ability to form viscous dispersions and/or gels when dispersed in water (Saha and Bhattacharya, J Food Sci Technol, 2010, 47(6):587-597). The extent of thickening varies with the type and nature of the hydrocolloid. Some provide low viscosities at a fairly high concentration, but most provide a high viscosity at a concentration below 1%. The viscosity of hydrocolloid dispersions arises predominantly from non-specific entanglement of conformationally disordered polymer chains. Hydrocolloids that can be used as thickening agents (referred to herein as hydrocolloid thickeners) include starch, modified starch, xanthan, galactomannans (such as guar gum, locust bean gum, and tara gum), gum Arabic or acacia gum, gum karaya, gum tragacanth, konjac maanan, and cellulose derivatives such as carboxymethyl cellulose, methyl cellulose, and hydroxypropylmethyl cellulose.

Some hydrocolloids are able to form gels, consisting of polymer molecules cross-linked to form an interconnected molecular network immersed in a liquid medium. A rheological definition of a gel is a viscoelastic system with a 'storage modulus' (G') larger than the 'loss modulus' (G") (de Vries 2004, Gums and stabilizers for the food industry, vol 12. RSC Publ, Oxford, pp 22-30). Hydrocolloids form gels by physical association of their polymer chains through hydrogen bonding, hydrophobic association, and cation-mediated cross-linking. Gelling-type hydrocolloids (or hydrocolloid gelling agents) include alginate, pectin, carrageenan, gelatin, gellan, agar, modified starch, methyl cellulose and hydroxypropylmethyl cellulose.

Gelation of hydrocolloids can occur by different mechanisms: ionotropic gelation, cold-set gelation and heat-set gelation (Burey et al. 2008, Crit Rev Food Sci Nutr 48:361-377). Ionotropic gelation occurs via cross-linking of hydrocolloid chains with ions, typically a cation-mediated gelation process of negatively-charged polysaccharides. Examples of hydrocolloids that can form gels by ionotropic gelation include alginate, carrageenan and pectin. Ionotropic gelation can be carried out by either diffusion setting or internal gelation. In cold-set gelation, hydrocolloid powders are dissolved in warm/boiling water to form a dispersion which forms a gel on cooling. Agar and gelatin form gels by this mechanism. Heat-set gels require the application of heat to gel (for example, curdlan, konjac glucomannan, methyl cellulose, starch and globular proteins).

Thus, in some embodiments, a composition of the invention comprises a viscosity-increasing agent, such as a thickener or gelling agent, for example a hydrocolloid. In particular embodiments, the hydrocolloid is, or comprises, a polysaccharide or a protein. The hydrocolloid may be a hydrocolloid thickener, such as starch, modified starch, xanthan, a galactomannan (such as guar gum, locust bean gum, and tara gum), gum Arabic or acacia gum, gum karaya, gum tragacanth, konjac maanan, or a cellulose derivative, such as carboxymethyl cellulose, methyl cellulose, or hydroxypropylmethyl cellulose.

In other embodiments, the hydrocolloid is, or comprises a cross-linked hydrocolloid, for example a cross-linked polysaccharide, such as cross-linked alginate, pectin, carrageenan, gelatin, gellan, agar, agarose, modified starch, or a cellulose derivative, such as methyl cellulose or hydroxypropylmethyl cellulose.

The hydrocolloid may be cross-linked by any suitable method, for example including the methods for gelation of hydrocolloids described above: ionotropic gelation, cold-set gelation and heat-set gelation. In particular embodiments, molecules of the hydrocolloid are cross-linked by cations (for example calcium ions) as a result of ionotropic gelation of a hydrocolloid gelling agent. Examples of hydrocolloid cross-linked by cations that may be present in a composition of the invention include alginate, carrageenan or pectin.

In particular embodiments, a composition of the invention includes cross-linked alginate, for example alginate cross-linked by calcium ions. Alginate can form gels without prior heating because sodium alginate is soluble in cold water.

Cross-linked alginate may be formed from sodium alginate and calcium ions (for example, provided by calcium chloride). In some embodiments, water may be used as solvent to dissociate the calcium ions. However, since this could potentially activate production of hydrogen peroxide by the enzyme and the substance that includes a substrate for the enzyme, and limit the stability of the composition, it may be preferred to use a non-aqueous solvent to dissociate the calcium ions, such as ethanol or acetic acid.

We have appreciated that glycerol may be used to bind free water. This property allows water to be used to dissolve the alginate, provided sufficient glycerol is present to prevent premature release of hydrogen peroxide from the enzyme and the substance that includes a substrate for the enzyme.

A method of making a composition may comprise mixing a lipophilic component, an aqueous component, a purified enzyme that is able to convert a substrate to release hydrogen peroxide, and a purified substrate for the enzyme.

A method of making a composition may comprise mixing a lipophilic component, an aqueous component, a purified enzyme that is able to convert a substrate to release hydrogen peroxide, and a purified precursor-substrate that can be converted to a substrate for the enzyme.

A method of making a composition may comprise mixing a first component (or liquid of a first phase), a second component (or liquid of a second phase), a purified enzyme that is able to convert a substrate to release hydrogen peroxide, and a purified substrate for the enzyme to form the composition, wherein the first component (or liquid of the first phase) and second component (or liquid of the second phase) are immiscible.

A method of making a composition may comprise mixing an oil, a purified enzyme that is able to convert a substrate to release hydrogen peroxide, and a purified substrate for the enzyme, to form the composition.

Methods of the invention may also comprise mixing a non-aqueous solvent, such as a polar, organic solvent.

A method of the invention may employ a rheometer to form compositions of the invention. A rheometer may allow for control of shear rate and temperature.

The enzyme and the substrate may be dissolved in a non-aqueous solvent to form a first mixture. The emulsifier may be added to the first phase, lipophilic phase or oil to form a second mixture. The first mixture may then be added dropwise to the second mixture to form an emulsion, whilst being mixed using e.g. a rheometer or mixer.

To form an emulsion, mixing may occur at a shear rate of between 1500 1/s to 2500 1/s, such as 2000 1/s. Mixing may occur at 30 and 50 °C, e.g. about 37°C.

A method of making a composition may comprise mixing the purified enzyme, the purified substrate for the enzyme, liquid of the second phase, liquid of the first phase, and optionally an emulsifier, under a high rate of shear for sufficient time to form an emulsion.

More stable emulsions may be formed if the ingredients of the emulsion are pre-mixed before contact with the emulsifier. Thus, in some embodiments, the enzyme, the substance that includes a substrate for the enzyme, liquid of the second phase, and liquid of the first phase are pre-mixed under a high rate of shear before contacting the pre-mixed ingredients with the emulsifier and mixing of the mixture comprising the pre-mixed ingredients and the emulsifier under the high rate of shear.

In some embodiments, the enzyme and the substrate are dissolved in the liquid of the second phase to form a solution before contacting the solution with the liquid of the first phase.

The high rate of shear may be from 1000 1/s to 4000 1/s. We have found that emulsions made using methods of the invention are more stable when formed using a higher rate of shear, for example from >2000 1/s to 4000 1/s, >2000 1/s to 3500 1/s, >2500 1/s to 4000 1/s, or >2500 1/s to 3500 1/s.

Mixing of the enzyme, the substance that includes a substrate for the enzyme, liquid of the second phase, liquid of the first phase, and the emulsifier (if present) may be carried out at a temperature of 20°C to 40⁰C, for example from 35°C to 40⁰C. More stable emulsions may be formed when mixing of the enzyme, the substance that includes a substrate for the enzyme, liquid of the second phase, liquid of the first phase, and the emulsifier (if present) is carried out at higher temperatures, for example from >37.5⁰C to 40⁰C, or 38⁰C to 40⁰C.

In some embodiments of methods of the invention, the enzyme, the substance that includes a substrate for the enzyme, liquid of the second phase, liquid of the first phase, and the emulsifier (if present), are mixed under a high rate of shear for at least 5 minutes, for example for 5 to 30 minutes.

Methods of the invention may be used to form creams, for example by inclusion of a viscosity-increasing agent, such as a thickener or gelling agent, for example a hydrocolloid.

In some embodiments, a method of the invention further comprises mixing a viscosity-increasing agent with the enzyme, the substance that includes a substrate for the enzyme, liquid of the second phase, liquid of the first phase, and the emulsifier (if present), under the high shear rate to form a cream.

In some embodiments, the viscosity-increasing agent is, or comprises a hydrocolloid, for example a polysaccharide.

In some embodiments, the hydrocolloid is, or comprises a hydrocolloid thickener, such as starch, modified starch, xanthan, a galactomannan (such as guar gum, locust bean gum, and tara gum), gum Arabic or acacia gum, gum karaya, gum tragacanth, konjac maanan, or a cellulose derivative, such as carboxymethyl cellulose, methyl cellulose, or hydroxypropylmethyl cellulose.

In some embodiments, the hydrocolloid is, or comprises a hydrocolloid gelling agent, such as alginate, pectin, carrageenan, gelatin, gellan, agar, agarose, modified starch, or a cellulose derivative, such as methyl cellulose or hydroxypropylmethyl cellulose.

The hydrocolloid gelling agent may capable of forming a gel by ionotropic gelation in the presence of cations. In such embodiments, a method of the invention further comprises mixing the cations with the hydrocolloid gelling agent, the enzyme, the substance that includes a substrate for the enzyme, liquid of the second phase, liquid of the first phase, and the emulsifier (if present), under the high shear rate to form the cream.

In some embodiments, the hydrocolloid gelling agent capable of forming a gel by ionotropic gelation, the enzyme, the substrate for the enzyme, liquid of the second phase, liquid of the first phase, and the emulsifier (if present), are mixed to form a mixture prior to contacting the cations with the mixture.

In some embodiments the hydrocolloid gelling agent capable of forming a gel by ionotropic gelation is contacted with the liquid of the second phase, the enzyme, and the substrate for the enzyme, prior to contact with the liquid of the first phase.

In some embodiments the cations are, or comprise calcium ions.

In particular embodiments the hydrocolloid gelling agent capable of forming a gel by ionotropic gelation in the presence of cations is, or comprises alginate, carrageenan or pectin, for example alginate.

In a particular embodiment, the hydrocolloid gelling agent is provided in aqueous solution, and the second phase is glycerol, wherein the glycerol is present in sufficient amount to bind free water in the composition and thereby prevent the enzyme catalysing release of hydrogen peroxide from the substrate for the enzyme.

In some embodiments the hydrocolloid gelling agent that is able to form a gel by ionotropic gelation (for example, alginate) is pre-mixed with the substrate for the enzyme, liquid of the second phase, and liquid of the first phase under a high rate of shear before the pre-mixed ingredients are contacted with the emulsifier (if present) and the cations (for example, calcium ions), and the mixture comprising the pre-mixed ingredients, the emulsifier (if present), and the cations is mixed under the high rate of shear.

The emulsifier (if present) may be contacted with the pre-mixed ingredients before the cations (for example, calcium ions). The cations (for example, calcium ions) may be added dropwise.

The cations (for example, calcium ions) may be provided in aqueous solution. Alternatively, the cations may be provided in non-aqueous solution, using a non-aqueous solvent, such as ethanol or acetic acid.

In particular embodiments, calcium chloride is provided in ethanol, and sodium alginate is provided in aqueous solution, and the second phase is glycerol, and the glycerol is present in sufficient amount to bind the free water in the alginate solution and thereby prevent the enzyme catalysing release of hydrogen peroxide from the substrate for the enzyme. This prevents premature release of hydrogen peroxide until the composition is contacted with water, thereby providing a stable composition.

As above, a method of the invention may employ a rheometer to form a composition of the invention. A rheometer may allow for control of shear rate and temperature. Alternatively, a high rate of shear may be provided by use of an ultrasonic probe, or a homogeniser.

Compositions of the invention may be sterilised by any suitable means. Preferably compositions of the invention have been sterilised by irradiation. The Applicant has found that compositions can retain glucose oxidase activity (and, therefore, the ability to release hydrogen peroxide on dilution) following sterilisation by exposure to gamma irradiation or electron beam irradiation. A suitable level of gamma irradiation is 10-70 kGy, preferably 25-70 kGy, more preferably 35-70 kGy. Alternatively, compositions of the invention may be sterilised by electron beam irradiation. A suitable level or dose of irradiation (e.g. electron beam irradiation) may be 10-100 kGy, preferably 30-80 kGy, more preferably 50-80kGy. The dose may be greater than 35 kGy. The dose may be less than 80 kGy, for example 75 kGy or less. In one embodiment, compositions of the invention may be sterilised by irradiation that is not gamma irradiation.

There is also provided according to the invention a method of sterilising a composition of the invention, which comprises exposing the composition to irradiation, preferably gamma irradiation or electron beam irradiation.

Since ozone has not been authorised by the US FDA for sterilisation of honey-based products for use in wound healing, compositions according to the invention preferably have not been sterilized by ozonation, and do not include ozone, or any components that have been subjected to sterilisation by ozonation. In particular, compositions according to the invention should not comprise ozonized honey or ozonated oil.

Preferred compositions for medical use according to the invention are sterile, single use compositions.

Sterilised compositions for use according to the invention that are stored away from exposure to light are expected to retain stability for at least six months. For example, such compositions may be packaged in high-density polyethylene/low-density polyethylene (HDPE/LDPE) tubes or in polyester-aluminium-polyethylene (PET/AI/PE) sachets.

Compositions of the invention may be in a container or sachet. The container may assist in maintaining the sterility of the composition. Preferably, the container is sealed or airtight. The container may have a removable and/or replaceable cap or seal. The container is preferably opaque.

A composition of the invention is preferably a medical grade or medical device grade composition. A composition of the invention may be a pharmaceutical grade composition.

A composition of the invention may be provided with a dressing. Suitable dressings include gauzes, bandages, tissues, films, gels, foams, hydrocolloids, alginates, hydrogels, or polysaccharide pastes, granules or beads. The composition may be present together with a wound-dressing matrix, such as a collagen or collagen-glycosaminoglycan matrix. The dressing may be a tulle dressing. Compositions in combination with a dressing are preferably sterile, and may be sterilised using irradiation, e.g. gamma irradiation.

According to the invention, there is provided a wound dressing comprising a dressing material for dressing a wound, and a composition of the invention.

According to the invention, the composition is in the form of a liquid preparation. Examples of liquid preparations include a syrup, paste, spray, drop, ointment, cream, lotion, oil, liniment, or gels. A typical gel includes an alcoholic gel such as an isopropanol, ethanol, or propanol gel, or a hydrogel.

A composition may be in a form suitable for administration to a human or animal subject. Suitable forms include forms adapted for topical or oral administration. Forms suitable for topical administration include a topical ointment, cream, lotion, oil, liniment, liquid, gel, or a dissolvable strip. Forms suitable for oral administration include a capsule, pellet, gel cap, pill, pillule, globule, lozenge, dental floss, toothpaste, mouthwash, dissolvable film strips. If a storage-stable composition is used, this may be diluted by liquid present at the site of administration (for example, by saliva for oral administration, or by exudate from a wound), leading to release of hydrogen peroxide at the administration site. According to the claimed invention, the composition is a liquid or gel composition.

Compositions of the invention can be used to treat any microbial infection that can be treated by hydrogen peroxide. Examples include infection caused by gram positive bacteria, gram negative bacteria, acid-fast bacteria, viruses, yeasts, parasitic or pathogenic micro-organisms or fungi. For example, infections caused by the following micro-organisms may be treated:
*Escherichia coli, Staphylococcus aureus, Pseudomonas aeruginosa, Candida albicans, Propionibacterium acnes, Staphylococcus aureus, Staphylococcus epidermidis, Staphylococcus saprophytics,* Beta haemolytic *Streptococci* Group A or B, *Campylobacter coli, Campylobacter jejuni,* Methicillin Resistant *Staphylococcus Aureus* (MRSA), Methicillin Sensitive *Staphylococcus Aureus* (MSSA), *Botrytis cinerea, Mycobacterium tuberculosis, Cryptosporidium, Plasmodium, Streptococcus pyogenes, Streptococcus zooepidemicus* and *Toxoplasma.*

There is further provided according to the invention a composition of the invention for use in the prevention or treatment of a microbial infection, for example a microbial infection that comprises a biofilm, or a microbe that is capable of forming a biofilm. So, there may be provided a compositions of the invention for use in the prevention or treatment of a microbial infection that comprises a biofilm or a microbe that is capable of forming a biofilm. The biofilm may comprise bacteria, fungi and/or viruses.

According to the invention there is also provided use of a composition or solution of the invention to prevent or inhibit microbial growth.

There is also provided according to the invention a composition of the invention for use as a medicament.

Compositions of the invention may be used to treat animals. Compositions of the invention may be used to treat humans.

There is further provided according to the invention a composition of the invention for the prevention, treatment, or amelioration of a microbial infection.

The subject may be a human or animal subject. Compositions of the invention may be topically administered.

Compositions of the of the invention may be for for use in a method of preventing or treating a sinus infection, such as CRS, which comprises administering an effective amount of a composition of the invention to a subject in need of such prevention or treatment.

To treat certain conditions, e.g. nasal infections such as CRS, a composition of the invention may be diluted such that the composition is at a particular treatment concentration. Certain treatment concentrations may be optimal for different conditions. For example, it has been found that compositions of the invention which contain 1000 ppm of glucose oxidase, 52 wt. % fructose, 31wt. % glucose and 17 wt.% 50 mMol Citric acid/NaOH buffer pH 7.04 may be optimal when forming an aqueous solution at a concentration of 71 g/l, particularly for treatment of microbes such as MRSA and MSSA, and biofilms which include such microbes. Compositions of the invention may thus be particularly effective against MRSA and MSSA.

Consequently, it may be beneficial to dilute compositions of the invention such that they form a solution of concentration of at least 30 g/l, such as from 30 g/l to 150 g/l. For example, the concentration of solution formed may be 50 to 100 g/l, such as 65 to 75 g/l.

The concentration of hydrogen peroxide produced in aqueous solutions formed by compositions of the invention may be at least 10 µM, such as 10 to 50 µM, for example 20 to 30 µM. The concentration may be maintained for at least an hour, preferably at least 2 hours, more preferably at least 10 hours and even more preferably at least 24 hours.

According to the invention, there is provided a method of forming an antimicrobial solution comprising diluting a composition of the invention in an aqueous solution such that there is sufficient free water to allow the enzyme to convert the substrate. The method may comprise diluting the composition to form a solution which contains at least 30 g/l of the composition. The method may comprise diluting the composition to form a solution which contains 30 g/l to 150 g/l, 50 to 100 g/l or 65 to 75 g/l of the composition. According to the invention, there may also be provided a solution obtained or obtainable by this method. In the solution, the hydrogen peroxide may be present at a concentration of 10 to 50 µM, preferably 20 to 30 µM. The hydrogen peroxide may be present at a concentration of at least 10µM. The hydrogen peroxide may be present at a concentration less than 100 µM. The concentration may be maintained for at least 1 hour, preferably at least 2 hours, more preferably at least 10 hours and even more preferably at least 24 hours.

Compositions or solutions of the invention may be for use in the treatment of a microbial infection that comprises a biofilm. The microbial infection may comprise *Haemophilus influenza,* MRSA or MSSA. The compositions or solutions may be for use in the treatment of chronic rhinosinusitis.

Compositions or mixtures of the invention may also be administered to the lungs to prevent or treat a microbial infection, for example a microbial infection that comprises a biofilm, or a microbe that is capable of forming a biofilm, in lung tissue. For example, compositions or mixtures of the invention may be administered to the lungs to prevent or treat tuberculosis, or to prevent or treat a microbial infection associated with cystic fibrosis (CF). *Mycobacterium tuberculosis* is the causative agent of tuberculosis. Compositions or mixtures of the invention may be used to prevent or treat respiratory infection, for example respiratory infection in a subject suffering from respiratory disease, such as COPD, cystic fibrosis, bronchiectasis, or asthma, or an HIV/AIDS-associated respiratory infection, or respiratory infection associated with terminal disease.

Compositions of the invention may be used to prevent or treat a microbial infection in a patient with cystic fibrosis, especially a microbial infection in a patient with cystic fibrosis that comprises a biofilm, or a microbe that is capable of forming a biofilm. The infection may be a pulmonary infection. For example, a composition of the invention may be used to prevent or treat a pulmonary *Pseudomonas aeruginosa* infection in a subject with cystic fibrosis.

Compositions of the invention may be used to treat a microbial lung infection, which comprises administering an effective amount of a composition of the invention to a subject in need of such prevention or treatment.

An example of a lung infection bronchiectasis. Respiratory tract infections associated with bronchiectasis include infections caused by the following bacteria: *Staphylococcus aureus, Haemophilus influenzae, Pseudomonas aeruginosa, Streptococcus pneumoniae,* non-tuberculous mycobacteria.

Compositions of the invention may be used to treat chronic obstructive pulmonary disease (COPD), which is an umbrella term for people with chronic bronchitis, emphysema, or both. *Enterobacteriaceae, P. aeruginosa* and *Staphylococcus aureus* have been implicated in COPD exacerbations. Various microbial pathogens have been implicated in chronic infection in COPD. These include typical bacteria such as non-typeable *H. influenzae* and *P. aeruginosa,* atypical bacterium such as *C. pneumoniae,* viruses such as adenovirus and possibly respiratory syncytial virus, and a fungus, *Pneumocystis jiroveci.*

Some of the most common opportunistic infectious lung diseases seen in HIV-positive or AIDS patients are *pneumocystis carinii pneumonia,* tuberculosis (caused by *Mycobacterium tuberculosis*), mycobacterium avium complex (*Mycobacterium avium-M. intracellulare* complex (MAIC)), fungal infections (such as candidiasis or coccidioidomycosis) and viral and bacterial pneumonia (such as bacterial pneumonia caused by *Haemophilus influenzae*)*.*

Compositions of the invention may be used for the prevention or treatment of infections, particularly respiratory infections, caused by any of the following microbes: *Pseudomonas aeruginosa; Staphylococcus aureus; Haemophilus influenza; Streptococcus pneumoniae,* non-tuberculous mycobacteria; *Enterobacteriaceae; C. pneumonia;* adenovirus; respiratory syncytial virus; *Pneumocystis jiroveci*; *pneumocystis carinii pneumonia*; *Mycobacterium tuberculosis*; *Mycobacterium avium-M. intracellulare* complex (MAIC); *candida*; *Coccidioides immitis; Mycobacterium abscessus.*

Compositions of the invention may be used to treat lower genital tract infections. For example, compositions of the invention may be applied topically to treat such infections. Examples of such infections include bacterial vaginosis and general bacterial vaginal discharge. Compositions of the invention may be applied to an insertion device, such as a tampon.

Compositions of the invention may be used to treat infections comprising Carbapenem-resistant enterobacteriaceae (CRE) or Carbapenemase-producing Enterobacteriaceae (CPE) bacteria.

Compositions of the invention may be used to treat urinary tract infections. For example compositions of the invention may be administered using a catheter. Compositions of the invention may be mixed with a saline solution prior to administration using a catheter. The compositions may be administered daily for, e.g., a period of at least one week, possibly up to a period of about two weeks.

Compositions of the invention may also be used to treat infections of other mucosal membranes, such as the bronchial mucosa and the lining of vocal folds, endometrium, esophageal mucosa, gastric mucosa, intestinal mucosa, nasal mucosa, olfactory mucosa, oral mucosa, penile mucosa, vaginal mucosa.

A composition of the invention may be administered or applied by spraying, by injection, by inhalation, or applying the composition to a patient's nasal cavity or paranasal sinus, or to a patient's anus or rectum. The composition may be administered or applied externally or internally, to a patient.

Compositions of the invention may be administered to sites such as the pleural cavity, for example in empyema. This may be achieved via chest drain instillation.

Compositions of the invention may be administered to the peritoneum, for example in complex infected intra-abdominal surgery. This may be achieved via surgical procedure or abdominal drain.

Compositions of the invention may be applied to prosthetic devices, such as orthopaedic prosthetic implants, e.g. prosthetic joints. This may assist in preventing infection following surgery.

Compositions of the invention may be provided in an inhaler, for example, a metered-dose inhaler, a dry powder inhaler, a nebulizer, for delivery of the composition into the lungs, or in a nasal inhaler.

Compositions of the invention may be sprayable or atomisable. For example, the composition may have rheological properties that permit spraying or atomisation.

There is also provided a device for delivering a composition to a patient, the device comprising a composition of the invention. Such device does not form part of the claimed invention.

The device may be a spraying or atomising device, such as a pump-action spray or an aerosol spray. The device may be an inhaler, for example, a metered-dose inhaler, a dry powder inhaler, a nebulizer, for delivery of the composition into the lungs, or a nasal inhaler.

A nebuliser is a device that converts liquid into aerosol droplets suitable for inhalation. Nebulisers use oxygen, compressed air or ultrasonic power to break up medication solutions and deliver a therapeutic dose of aerosol particles directly to the lungs. A wide variety of nebulisers is available. Nebulisers can be driven by compressed gas (jet nebuliser) or by an ultrasonically vibrating crystal (ultrasonic nebuliser).

In order to produce small enough particles from solution in 5-10 minutes, gas flow rates of at least 6 L/minute are usually necessary. Ultrasonic nebulisers use a rapidly vibrating piezoelectric crystal to produce aerosol particles. Ultrasonic nebuliser machines are often smaller and quieter.

Many nebulisers deliver only 10% of the prescribed drug dose to the lungs. Much of the drug is caught on the internal apparatus or wasted during exhalation. The efficiency of drug delivery depends on the type and volume of nebuliser chamber and the flow rate at which it is driven. Some chambers have reservoir and valve systems to increase efficiency of particle delivery during inspiration and reduce environmental losses during expiration. Breath-assisted open vent systems improve drug delivery but are dependent on the patient having an adequate expiratory flow. Face masks or mouthpieces may be used for administration of aerosol particles.

Nebulisers are used for the treatment of many respiratory diseases. Indications for nebuliser use include the management of exacerbations and long-term treatment of chronic obstructive pulmonary disease (COPD), management of cystic fibrosis, bronchiectasis, asthma, HIV/AIDS and symptomatic relief in palliative care.

Nebulised compositions of the invention may be used to prevent or treat a microbial infection, for example a microbial infection that comprises a biofilm, or a microbe that is capable of forming a biofilm, in a subject suffering from respiratory disease, such as COPD, cystic fibrosis, bronchiectasis, or asthma, or an HIV/AIDS-associated respiratory infection, or respiratory infection associated with terminal disease.

The device may be for external use, such as for applying the composition to a patient's skin.

The device may be for internal application to a patient. For example, the device may be an inhaler or nebuliser for administering the composition to the patient's respiratory tract. The device may be a douche. The device may be a device for injecting the composition into the patient, such as a syringe. Compositions of the invention may thus be injectable. Methods of the invention may comprise injecting compositions of the invention into a patient.

Compositions of the invention may be for prophylactic applications.

For example, there may be provided a prophylactic spray for pre-operation skin sterilisation. Such a spray may comprise a low-viscosity composition. When applied, the composition may be non-sticky. The spray may allow delivery of a reactive oxygen laminar to the skin. The laminar may continue to deliver reactive oxygen over an extended period of time which could sterilise skin prior to surgical incision and provide prophylactic protection during and after the surgical procedure.

There may be provided an atomising spray for inhalation. An atomising spray for inhalation may be used to treat infections in a patient's airways. An atomising spray may require a composition with low viscosity.

A spray could be used to apply to burn or wound tissue prior to applying a dressing. This may require a more viscous composition.

A spray could be applied internally to a patient, post-surgery, to prevent organ infection and septicaemia. This may require a more viscous composition.

A composition of the invention may be contained within a water-soluble container, such as a sachet or pouch.

So, there may be provided a water-soluble container enclosing, or containing, a composition of the invention.

Advantageously, this may allow a precise amount of the composition to be delivered for a particular therapeutic application by adding a measured amount of solvent, such as water or saline. For example, a composition of the invention contained in a water-soluble sachet may be used to form a nasal douche solution, or a solution that is to be nebulised or atomised. Consequently, contacting the soluble sachet containing the composition, with an aqueous solvent, may result in formation of an aqueous mixture of the invention. The aqueous mixture may contain sufficient free water to allow the enzyme to convert the substrate and produce hydrogen peroxide.

The water-soluble sachet is preferably manufactured from a medical grade material. The sachet may be dissolvable in water at 38°C. Preferably, the water-soluble sachet is non-toxic, anti-static, resistant to degradation by ultraviolet light and resistant to degradation by gases, oils and greases. In one example, the soluble sachet may be manufactured from a polymer or plastics material, such as polyvinyl alcohol.

Compositions of the invention contained in water-soluble sachets may be useful for treating one or more of the following conditions: nasal conditions, such as sinusitis and rhinosinusitis; respiratory tract infections, such as upper respiratory tract infections (e.g. tonsillitis, laryngitis and sinusitis or lower respiratory tract infections (e.g. bronchitis, pneumonia, bronchiolitis and tuberculosis); chronic obstructive pulmonary disease; and cystic fibrosis.

There may be provided a wound dressing which comprises a composition of the invention, and a water-soluble material which may function as a barrier or layer. The water-soluble material may be in contact with, or adjacent to, the composition. The composition of the invention may be enclosed by, or contained within, the water-soluble material. The water-soluble material may thus form a sachet, pouch or enclosure. The composition may be positioned between layers of the water-soluble material. The water-soluble material may be manufactured from a medical grade material. The water-soluble material may be dissolvable in water at 38°C. Preferably, the water-soluble material is non-toxic, anti-static, resistant to degradation by ultraviolet light and resistant to degradation by gases, oils and greases. In one example, the water-soluble material may be manufactured from a polymer or plastics material, such as polyvinyl alcohol. In use, the dressing may be applied to a wound, and the water-soluble material may thus dissolve in wound exudate, allowing the composition of the invention to contact the wound and deliver reactive oxygen to the wound. Advantageously, a measurable and accurate dose of reactive oxygen may thus be delivered to a wound. The water-soluble material may control release of the composition from the wound dressing.

The water-soluble material and the composition may be attached to the surface of a conventional aseptic wound dressing.

Compositions of the invention with a high osmolarity (suitably having a water activity (a_{w}) in the range similar to honey, i.e. 0.47-0.7) are believed to facilitate the debridement of wounds by the autolytic action of tissue proteases. They may create a moist wound environment by drawing out lymph fluid from the wound tissues through their strong osmotic action. This provides a constant supply of proteases at the interface of the wound bed and the overlying necrotic tissue. This action also washes the surface of the wound bed from beneath. The debriding action may also contribute to the lowering of a wound's bacterial load by removal of dead tissue. Dead tissue is well known to provide an excellent medium for bacterial growth and increase the risk of infections if left in the wound.

A composition of the invention may be used in a method of wound care, including the treatment of a wound, or the treatment or management of wound sepsis.

The wound may be an acute wound, chronic wound, surgical wound (for example, a Caesarean wound), chronic burn, or an acute burn. A composition of the invention may be used in the prophylactic prevention of wound sepsis. If a storage-stable composition of the invention is used, it will be appreciated that this may be diluted by liquid present at the wound site, which thereby leads to the release of hydrogen peroxide by the diluted composition.

There is also provided according to the invention a composition of the invention for treatment of a wound.

Compositions of the invention may be used to treat wounds that are critically colonized. The term "critically colonized" is often used to refer to a wound that has reached a critical point at which bacteria begin to negatively affect the wound and begin to elicit signs of their presence. A critically colonized wound may indicate the presence of a biofilm. A bacterial load of greater than 10⁵ organisms/gram of tissue is often accepted as impeding wound healing (Siddiqui AR, Bernstein JM (2010) Chronic wound infection: Facts and controversies. Clinics in Dermatology 28: 519-26; Edmonds, M., & Foster, A. (2004). The use of antibiotics in the diabetic foot. Am J Surg, 187(5A), 25S-28S. Consequently, compositions of the invention may be used to treat wounds that have a bacterial load of greater than 10⁵ organisms/gram of tissue.

There is also provided according to the invention a method of treating inflammation, which comprises administering a composition of the invention to a site of inflammation.

A composition of the invention may be for use in treatment of inflammation.

A composition of the invention may be for use in stimulating tissue growth.

A composition of the invention may be for use in debriding a wound.

A composition of the invention may be for use in deodorising a wound.

For wound healing applications, compositions of the invention may be administered at an appropriate frequency determined by the healthcare provider. Suitably compositions of the invention may be administered at least every several days, for example every week, but preferably every day or every other day.

The amount of a composition of the invention administered will depend on many factors, such as the strength of the antimicrobial properties of the composition, and other wound healing properties of the composition, on the size of the wound, and on the age and condition of the subject to be treated. However, for many applications it is expected that administration of 2-100g, or 5-100g of a composition of the invention will be suitable, preferably 10-50g.

A composition of the invention may be in a form suitable for controlled or sustained-release delivery. For example, an oral administration form may have an enteric coating to provide for controlled or sustained-release delivery.

A composition of the invention may be in a form for use as a cosmetic composition. A composition of the invention may be present with at least one suitable cosmetic excipient or adjuvant. Cosmetic applications cover many different personal care applications, including the treatment of hair conditions, such as dandruff, or the treatment of body odour.

A composition of the invention may be provided in the form of a prophylactic hand barrier solution or hand sanitizer solution. Such a hand barrier solution may be provided in the form of a cream, lotion, or hydrogel, and is used as a hand wash type product with advantageous properties for the prophylactic prevention of microbial infection. A composition of the invention may be administered as part of a tissue or skin wipe.

The microbial infection may be an oral, eye, ear, skin, chest, or nail infection. The oral infection may be gum disease, oral ulceration and/or an oral hygiene disorder. The oral hygiene disorder may be halitosis and/or gingivitis. Alternatively, the oral infection may be a throat infection or a nasal infection, including nasal Staphylococci infections.

Compositions of the invention may be used to treat conditions that may not be the result of an infection by a pathogen, such as a bacterium, virus or fungus. Such conditions include oral conditions.

For example, aphthous ulcers, such as recurrent aphthous ulcers, may not result from infection by a pathogen. Aphthous ulcers are characterized by the repeated formation of benign and non-contagious mouth ulcers (aphthae) in otherwise healthy individuals The cause of aphthous ulcers is not completely understood, but is believed to involve a T cell-mediated immune response triggered by a variety of factors. Triggers may include nutritional deficiencies, local trauma, stress, hormonal influences, allergies, genetic predisposition or other factors

Another oral condition that may not result from infection of a pathogen is geographic tongue. This is an inflammatory condition of the mucous membrane of the tongue, usually on the dorsal surface.

Dentists typically find it difficult to treat such conditions.

A composition as described herein may be for use in the treatment of a condition that does not the result from an infection by a pathogen. The condition is preferably an oral condition.

A composition as described herein may be for use in the treatment of aphthous ulcers (preferably recurrent aphthous ulcers) or geographic tongue.

Compositions may be administered topically. Compositions may be administered directly to the affected site in a subject's mouth.

An eye infection may include conjunctivitis. The skin infection may be a fungal skin infection. Fungal skin infections include athlete's foot and/or ringworm in humans. In veterinary medicine, fungal skin conditions include foot rot, ringworm and the control of zoonotic skin infections. The nail infection may be a fungal nail infection, such as onychomycosis.

A composition of the invention may be used for the treatment of a skin disorder, such as acne, eczema, or psoriasis. Acne and eczema may have a microbial infection component which can be treated by the composition, and secondary microbial infections of psoriatic lesions caused by scratching can be treated by a composition of the invention.

A composition of the invention may be used in veterinary medicine. Important veterinary applications include the treatment of microbial infections and the treatment or management of wound care, or burn treatment. Specific conditions include chronic skin infections in dogs (subcutaneous Staphylococcus infections), Otitis externa (ear infections), oral care in animals, Campylobacter infections in chickens, coliosis, enteric microbial infections in pigs, poultry and cattle, Cryptosporidium infections, clearance of zoonotic infections, wound dressing, e.g. horn removal, and abscess treatment. The present invention has particular advantages in veterinary usage, in that it allows the treatment of microbial infections without introducing antibiotics into the food chain.

A composition of the invention may be provided with any other composition or product for which it is desired to provide the ability to generate antimicrobial activity. The composition of the invention may be provided as a mixture with the other composition or product, or separately therefrom, for example packaged as a kit with the other composition or product. Where a composition of the invention is provided as a mixture with the other composition or product, it is preferred that this other composition or product does not include sufficient free water to allow the enzyme to convert the substrate of the composition of the invention.

A composition of the invention may be provided with instructions for use of the composition. For example, a composition of the invention may be packaged as a kit with the instructions.

According to the invention, there are also provided methods for making compositions of the invention.

A method for producing a composition of the invention, may comprise contacting an enzyme that is able to convert a substrate to release hydrogen peroxide, with the substrate.

Preferably, the enzyme and substrate are purified, as described herein.

The enzyme and substrate, or enzyme may be contacted or mixed in a dry or solid form.

The method may comprise mixing a solute, the solute as described herein.

The method may comprise dissolving the substrate in a solvent, such as water, before adding enzyme.

According to the invention, there is provided a method for producing a liquid or gel composition for generating antimicrobial activity, which comprises: contacting a purified enzyme that is able to convert a substrate to release hydrogen peroxide with a purified substrate for the enzyme, a solute in the form of a sugar, the solute having a solubility greater than 100g/100g water at 20°C and 1 atm, and a polymer, wherein the composition has a water activity less than 0.6 and substantially no hydrogen peroxide.

In some embodiments, to form a composition of the invention, fructose and glucose are dissolved in water. Fructose may be dissolved at room temperature, whereas glucose may require heating and stirring to dissolve.

According to the invention, there is provided a method of forming an antimicrobial solution, the method comprising contacting, or diluting, a composition of the invention with sufficient water such that the enzyme is able to convert the substrate to generate hydrogen peroxide, and optionally such that the precursor-substrate is able to be converted to the substrate .

There may be provided a kit comprising a first composition and a second composition, in which the first composition comprises an enzyme that is able to convert a substrate to release a hydrogen peroxide, the second composition comprises a substrate for the enzyme, and in which the first and second compositions are separate.

The first composition does not comprise the substrate for the enzyme and the second composition does not comprise the enzyme for the substrate. Consequently, hydrogen peroxide production may only be initiated once the first and second compositions have been mixed. The first and second compositions may be aqueous solutions. The kit may contain a pre-determined amount of enzyme, substrate and solvent such that hydrogen peroxide production can proceed, when the first and second compositions are mixed, at a desirable level (as described herein). Compositions of the kits of the invention may have any of the further features of the compositions of the invention as described herein. For example, the enzyme is preferably purified and the substrate is preferably purified (as described herein). Preferably, the first and second compositions of the kit are sterile, as described herein.

Example are now described, by way of example only, with reference to the accompanying drawings in which:
Figure 1 is a graph showing the effect of compositions of the invention comprising glucose, glucose oxidase and fructose (SyntheticRO) on the growth of planktonic MRSA, compared to Surgihoney^{™}, at various concentrations.
Figure 2 is a graph showing the effect of sterile and non-sterile compositions of the invention comprising glucose, glucose oxidase and fructose (buffered at pH 4.03) on the growth of planktonic MRSA, at various concentrations.
Figure 3 is a graph showing the effect of sterile and non-sterile compositions of the invention comprising glucose, glucose oxidase and fructose (unbuffered) on the growth of planktonic MRSA, at various concentrations.
Figure 4 is a graph showing the effect of sterile and non-sterile compositions of the invention comprising glucose, glucose oxidase and fructose (buffered at pH 7.04) on the growth of planktonic MRSA, at various concentrations.
Figure 5 is a table showing the effect of sterile and non-sterile compositions of the invention comprising glucose, glucose oxidase and fructose, on the MIC and MBC of planktonic MRSA, at various concentrations.
Figure 6 shows an optical microscopy images of reverse micelles in an emulsion containing Surgihoney^{™}.

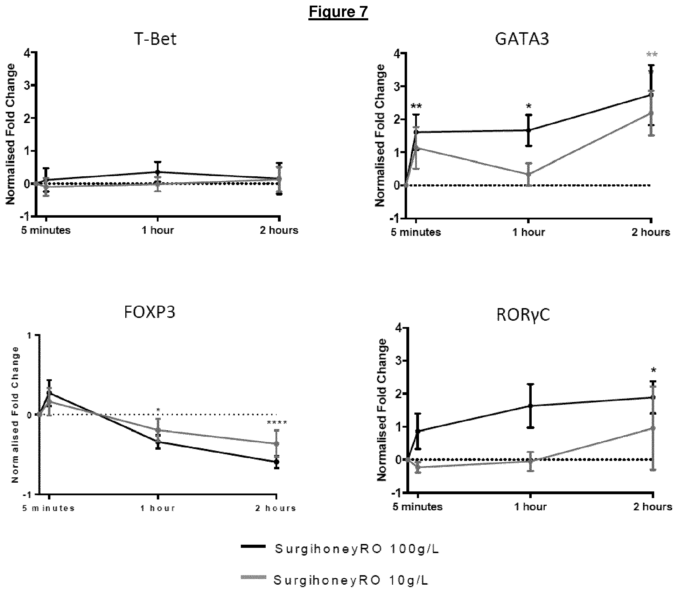

Figure 7 shows that SurgihoneyRO influences the balance of T-helper cell (Th) subsets by altering the expression of gatekeeping genes. Gene-expression profiles for Th lineage gatekeeping genes were analysed in nasal epithelial cells treated with 10g/L or 100g/L of SurgihoneyRO for 5 minutes, 1 hour and 2 hours using quantitative real-time PCR. The fold change was calculated based on normalisation to the expression of the endogenous ß-actin housekeeping gene. Each point shows the mean fold change in gene expression for 6 independent experiments. The dotted line for y=0 represents the untreated control group to which gene expression in the treatment groups were normalised and compared using an unpaired two tailed T test. *p ≤ 0.05, ** p ≤ 0.001 , *** p ≤ 0.001 , **** p ≤ 0.0001 ;

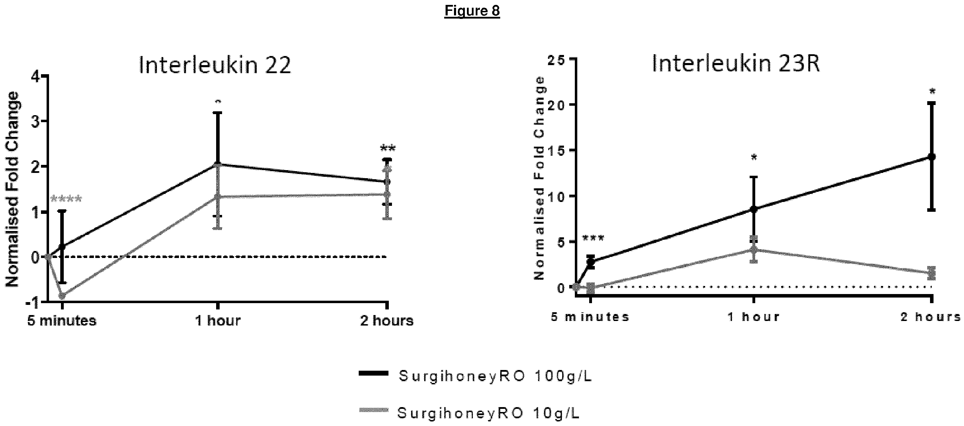

Figure 8 shows that SurgihoneyRO induces a Th₁₇ response in nasal epithelial cells. Gene-expression profiles for Th₁₇ related cytokines were analysed in the nasal epithelial cells treated with 10g/L or 100g/L of SurgihoneyRO for 5 minutes, 1 hour and 2 hours using quantitative real-time PCR. The fold change was calculated based on normalisation to the expression of the endogenous ß-actin housekeeping gene. Each point shows the mean fold change in gene expression for 6 independent experiments. The dotted line for y=0 represents the untreated control group to which gene expression in the treatment groups were normalised and compared using an unpaired two tailed T test. *p ≤ 0.05, ** p ≤ 0.001, *** p ≤ 0.001, **** p ≤ 0.0001;

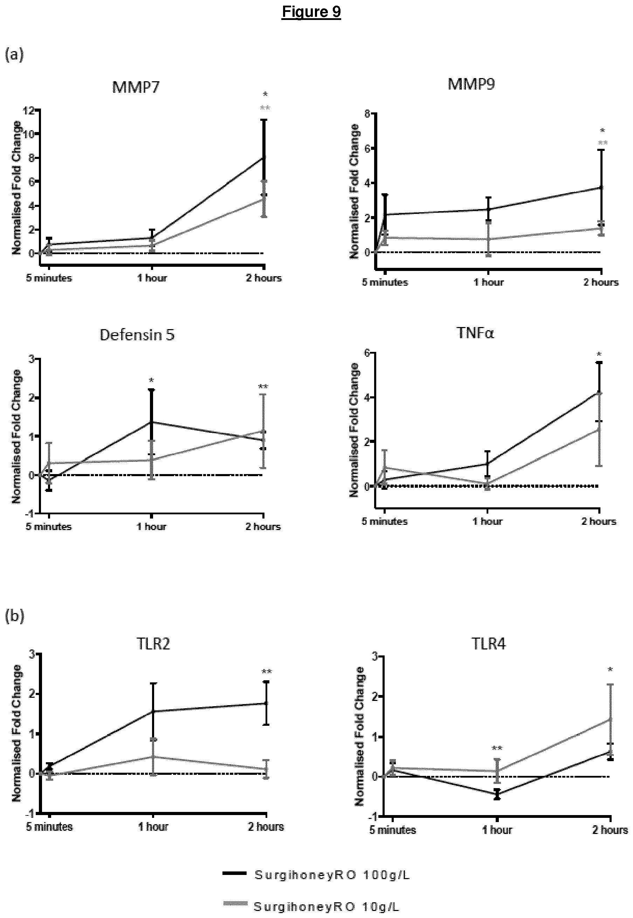

Figure 9 shows that SurgihoneyRO can modulate the host immune response to invading pathogens in nasal epithelial cells. Gene-expression profiles for (a) matrix metalloprotease (MMP) related genes and (b) toll-like receptor (TLR) genes were analysed in nasal epithelial cells treated with 10g/L or 100g/L of SurgihoneyRO for 5 minutes, 1 hour and 2 hours using quantitative real-time PCR. The fold change was calculated based on normalisation to the expression of the endogenous ß-actin housekeeping gene. Each point shows the mean fold change in gene expression for 6 independent experiments. The dotted line for y=0 represents the untreated control group to which gene expression in the treatment groups were normalised and compared using an unpaired two tailed T test. *p ≤ 0.05, ** p ≤ 0.001, *** p ≤ 0.001, **** p ≤ 0.0001 ;

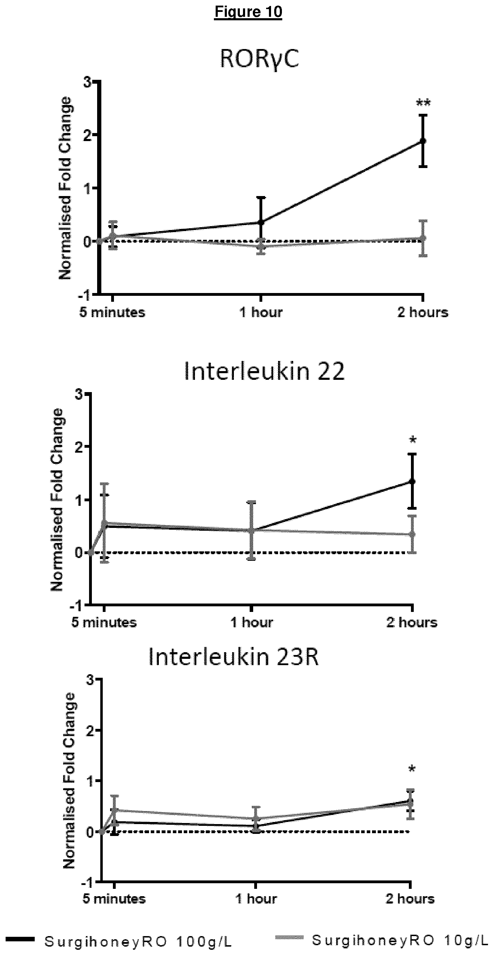

Figure 10 shows that SurgihoneyRO induces a Th₁₇ response in mast cells. Gene-expression profiles for Th₁₇ related cytokines were analysed in HMC-1 treated with 10g/L or 100g/L of SurgihoneyRO for 5 minutes, 1 hour and 2 hours using quantitative real-time PCR. The fold change was calculated based on normalisation to the expression of the endogenous ß-actin housekeeping gene. Each point shows the mean fold change in gene expression for 6 independent experiments. The dotted line for y=0 represents the untreated control group to which gene expression in the treatment groups were normalised and compared using an unpaired two tailed T test. *p ≤ 0.05, ** p ≤ 0.001 , *** p ≤ 0.001 , **** p ≤ 0.0001;
Figure 11 shows fluorimetric measurements of hydrogen peroxide. The concentration of hydrogen peroxide production was measured over a time course of 24 hours. The kinetics of hydrogen peroxide production by both SurgihoneyRO and Acacia honey were measured without the presence of cells (a), with nasal epithelial cell line (b) and with mast cell line (c). The data represents the mean with the error bars showing standard deviation of the mean for at least 3 independent experiments;

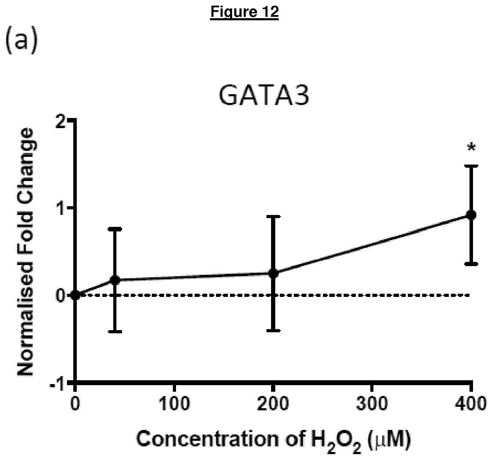

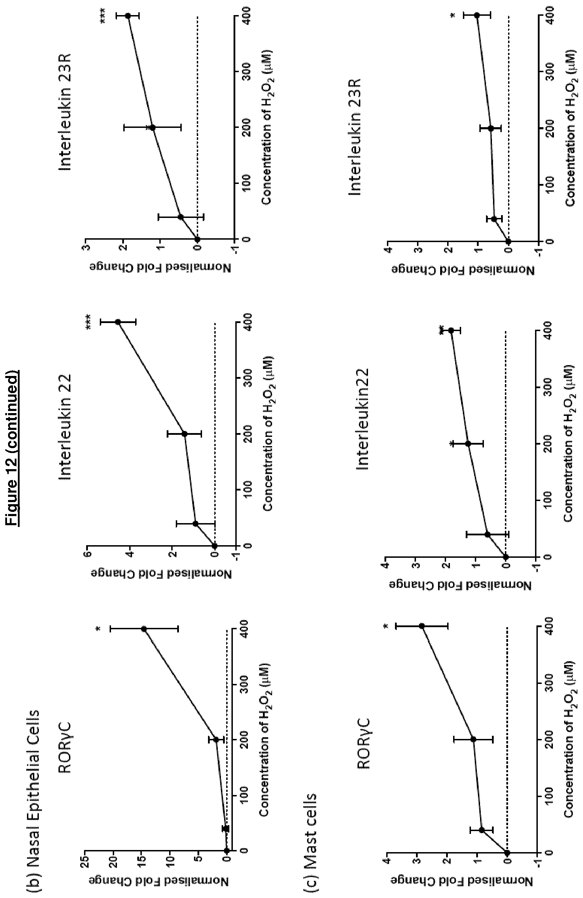

Figure 12 shows that hydrogen peroxide treatment influences the balance of T-helper cell (Th) subsets by altering the expression of the gatekeeping genes in the same way as SurgihoneyRO. Gene-expression profiles for Th lineage gatekeeping genes were analysed by RT-PCR. (a) shows GATA3 gene expression in the nasal epithelial cells, (b) shows the expression of Th₁₇ related cytokines for the nasal epithelial cells and (c) for the mast cells. Both cell lines were treated either with or without hydrogen peroxide (0-400µM) for 1 hour. The fold change calculated was based on normalisation to the expression of the endogenous β-actin housekeeping gene. Each point shows the mean fold change in gene expression for 5 independent experiments. The dotted line for y=0 representing the untreated control group to which gene expression in the treatment groups were normalised and compared using an unpaired two tailed T test. *p ≤ 0.05, ** p ≤ 0.001 , *** p ≤ 0.001 , **** p ≤ 0.0001;

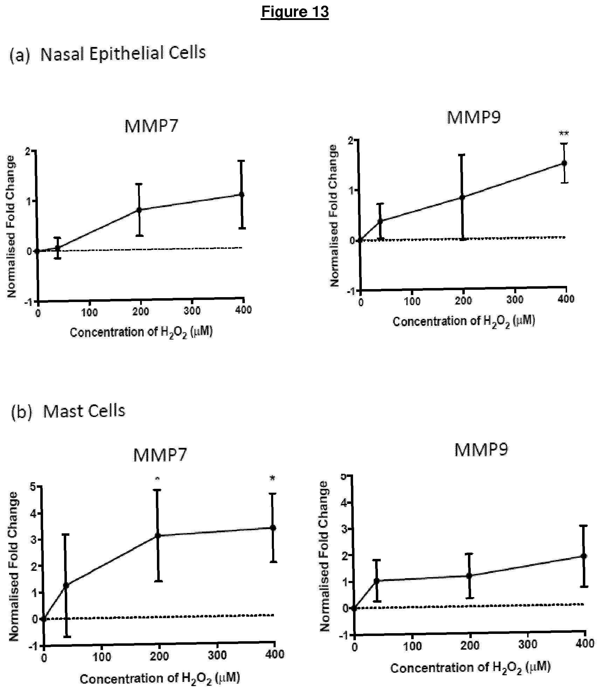

Figure 13 shows that hydrogen peroxide treatment can modulate anti-microbial and host immune response to invading pathogens in both nasal epithelial cells and mast cells. Gene-expression profiles for MMP related genes were analysed in (a) nasal epithelial cells and (b) mast cells treated with various concentrations of exogenous hydrogen peroxide (0-400µM) for 1 hour using quantitative real-time PCR. The fold change was calculated based on normalisation to the expression of the endogenous ß-actin housekeeping gene. For each treatment group the mean fold change in gene expression for 5 independent experiments is shown. The dotted line for y=0 representing the untreated control group to which gene expression in the treatment groups were normalised and compared using an unpaired two tailed T test. *p ≤ 0.05, ** p ≤ 0.001, *** p ≤ 0.001 , **** p ≤ 0.0001;

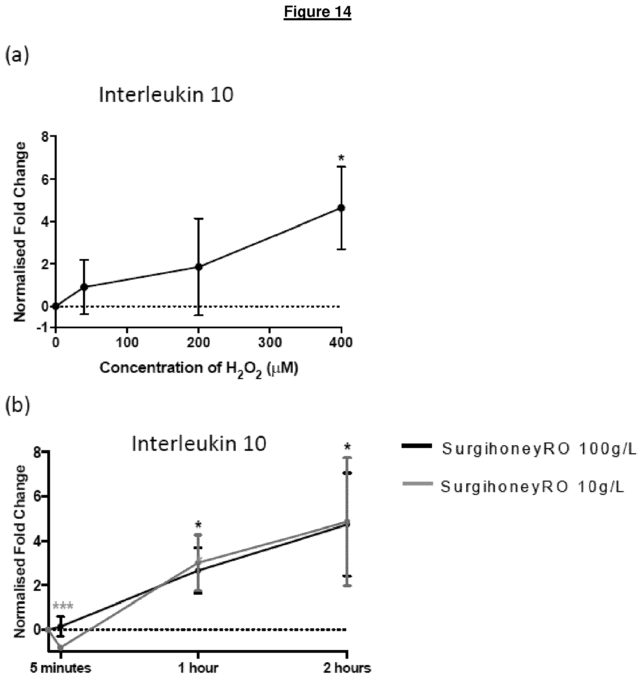

Figure 14 shows analysis of gene-expression of IL10 in the nasal epithelial cells treated with various concentrations of exogenous hydrogen peroxide (0-400µM) for 1 hour (a); with 10g/L or 100g/L of SurgihoneyRO for 5 minutes, 1 hour and 2 hours using quantitative real-time PCR. The fold change was calculated based on normalisation to the expression of the endogenous ß-actin housekeeping gene. Each point shows the mean fold change in gene expression for 5 independent experiments. The dotted line for y=0 representing the untreated control group to which gene expression in the treatment groups were normalised and compared using an unpaired two tailed T test. *p ≤ 0.05, ** p ≤ 0.001, *** p ≤ 0.001, **** p ≤ 0.0001;
Figure 15 shows the effect of compositions of the invention comprising glucose, glucose oxidase and fructose (SyntheticRO) on the growth of planktonic MRSA, compared to SurgihoneyRO, at various concentrations;

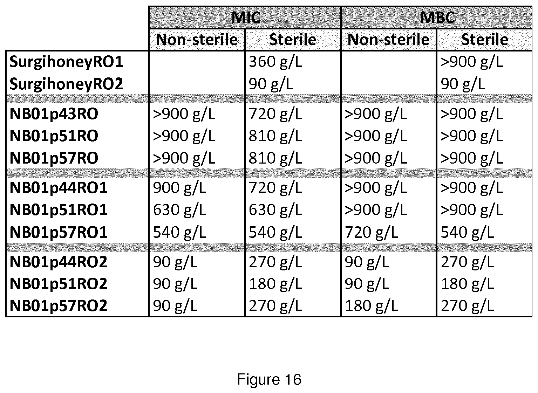

Figure 16 shows the effect of SyntheticRO on the MIC and MBC of planktonic MRSA, compared to SurgihoneyRO, at various concentrations;
Figure 17 shows the effect of SyntheticRO comprising glucose, glucose oxidase and fructose (SyntheticRO) on the growth of planktonic MSSA isolate;
Figure 18 (a and b) compares SyntheticRO with SurgihoneyRO using planktonic MRSA and MSSA, and Figure 18c is a table showing the MICs of a composition of the invention compared to SurgihoneyRO. Planktonic MRSA and MSSA *in vitro* cultures were grown in the presence of the respective compositions for 18 hours, then the absorbance (OD₅₉₅) measured and compared to untreated cultures (n=6);
Figure 19 shows 48 hour MRSA (a&c) and MSSA (b&d) *in vitro* biofilms treated with SyntheticRO+ (with enzyme) and SyntheticRO- (without enzyme) for 24 hours (n=5), with viability measured by cfu enumeration (a&b) and biomass measured by absorbance [OD₅₉₅] (c&d);
Figure 20 shows 48 hour MRSA and MSSA biofilms treated with 71 g/l SyntheticRO+ and SyntheticRO- for 24 hours and then imaged using confocal microscopy and LIVE/DEAD staining (b), with maximum biofilm height measured and compared to untreated controls (a);
Figure 21 shows 48 hour biofilms formed by individual MRSA (a; n=7) and MSSA (b; n=5) clinical isolates treated with 71 g/l synthetic RO+ and viability measured by cfu enumeration;

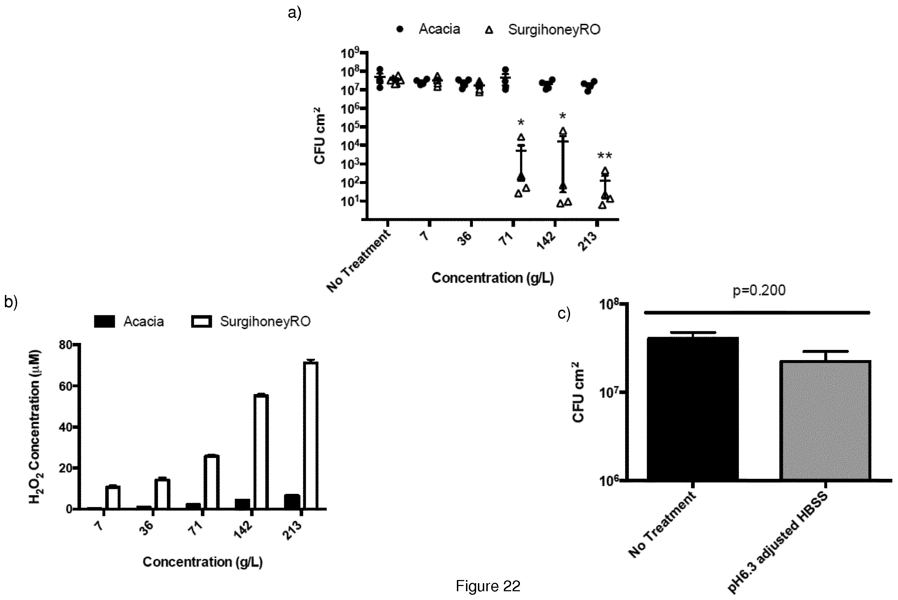

Figure 22 shows the result of Surgihoney treatment of *in vitro* non-typeable *H. influenza* biofilms. (a) NTHi in vitro planktonic cultures were grown in the presence of SurgihoneyRO or Acacia for 18 h then growth assessed by measurement of absorbance (OD₅₉₅). (b) 48 hour in vitro NTHi biofilms were treated with SurgihoneyRO or Acacia for 2 h and biofilm viability measured by cfu enumeration, (c) Fluorimetric measurements of H₂O₂ production by different SurgihoneyRO and Acacia concentrations at 2 h. (d) 48 hour in vitro NTHi biofilms treated with HBSS adjusted to pH6.3 (equivalent pH to 71 g/L SurgihoneyRO) for 2 h and biofilm viability measured by cfu enumeration *P≤0.05; **P≤0.01; and

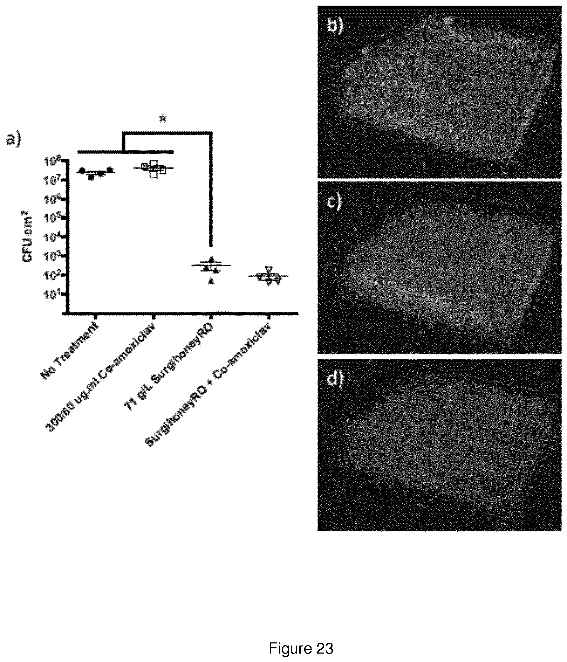

Figure 23 Shows a comparison between SurgihoneyRO and co-amoxiclav in the treatment of non-typeable H. influenza biofilms. (a) 48 hour in vitro NTHi biofilms were treated with 71g/L SurgihoneyRO and 300/60 µg.ml co-amoxiclav alone, and in combination for two hourswith viability measured by cfu enumeration. Confocal microscopy was performed on (b) untreated 48 h NTHi biofilms, biofilms treated for 2 hours with (c) 300/60 µg.ml co-amoxiclav and d) 71 g/: SurgihoneyRO. Biofilms were imaged using Live/Dead staining to visualize ive cells (green fluorescence) and dead cells (red fluorescence). *P≤0.05.

Surgihoney may also be referred to as Surgihoney^{™}, SurgihoneyRO or SurgihoneyRO^{™}. Compositions of the invention, such as compositions which comprise purified glucose, purified fructose and glucose oxidase may be referred to as SyntheticRO, synthetic honey compositions or synthetic compositions.

### Specific Examples

### Example 1 - Synthetic Honey Compositions (reference Example)

Samples with batch number "RO" contain no glucose oxidase.

Samples with batch number "RO1" contain 50 ppm glucose oxidase.

Samples with batch number "RO2" contain 1000 ppm glucose oxidase.

### A. pH 4.03 buffered samples

### A1. Batch no NB01p43RO

### Non sterile

| Material | Weight fraction |
|---|---|
| Fructose | 52.0% |
| Glucose | 31.0% |
| 50 mMol Citric acid/NaOH buffer pH 4.03 1 | 17.0% |

### Description

### Non sterile base buffered saccharide solution.

### A2. Batch no NB01p43RO

### Sterile

| Material | Weight fraction |
|---|---|
| Fructose | 52.0% |
| Glucose | 31.0% |
| 50 mMol Citric acid/NaOH buffer pH 4.03 | 17.0% |

### Description Sterile base buffered saccharide solution

### A3. Batch no NB01p44RO1

### Non sterile

| Material | Weight fraction |
|---|---|
| Fructose | 52.0% |
| Glucose | 31.0% |
| 50 mMol Citric acid/NaOH buffer pH 4.03 | 17.0% |

### Description

### Non sterile base buffered RO1 saccharide solution.

### A4. Batch no NB01p44RO1

### Sterile

| Material | Weight fraction |
|---|---|
| Fructose | 52.0% |
| Glucose | 31.0% |
| 50 mMol Citric acid/NaOH buffer pH 4.03 | 17.0% |

### Description

### Sterile base buffered RO1 saccharide solution

### A5. Batch no NB01p44RO2

### Non sterile

| Material | Weight fraction |
|---|---|
| Fructose | 52.0% |
| Glucose | 31.0% |
| 50 mMol Citric acid/NaOH buffer pH 4.03 | 17.0% |

### Description

### Non sterile base buffered RO2 saccharide solution.

### A6. Batch no NB01p43RO2

### Sterile

| Material | Weight fraction |
|---|---|
| Fructose | 52.0% |
| Glucose | 31.0% |
| 50 mMol Citric acid/NaOH buffer pH 4.03 | 17.0% |
| GOX enzyme | N/A |

### Description Sterile base buffered RO2 saccharide solution

### B. Unbuffered samples

### B1. Batch no NB01p51RO

### Non sterile

| Material | Weight fraction |
|---|---|
| Fructose | 52.0% |
| Glucose | 31.0% |
| Water | 17.0% |

### Description

### Non sterile base buffered saccharide solution.

### B2. Batch no NB01p51RO

### Sterile

| Material | Weight fraction |
|---|---|
| Fructose | 52.0% |
| Glucose | 31.0% |
| Water | 17.0% |

### Description Sterile base buffered saccharide solution

### B3. Batch no NB01 p51 RO1

### Non sterile

| Material | Weight fraction |
|---|---|
| Fructose | 52.0% |
| Glucose | 31.0% |
| Water | 17.0% |

### Description

### Non sterile base buffered RO1 saccharide solution.

### B4. Batch no NB01p51RO1

### Sterile

| Material | Weight fraction |
|---|---|
| Fructose | 52.0% |
| Glucose | 31.0% |
| Water | 17.0% |

### Description

### Sterile base buffered RO1 saccharide solution

### B5. Batch no NB01 p51 RO2

### Non sterile

| Material | Weight fraction |
|---|---|
| Fructose | 52.0% |
| Glucose | 31.0% |
| Water | 17.0% |

### Description

### Non sterile base buffered RO2 saccharide solution.

### B6. Batch no NB01p51 RO2

### Sterile

| Material | Weight fraction |
|---|---|
| Fructose | 52.0% |
| Glucose | 31.0% |
| Water | 17.0% |

### Description

### Sterile base buffered RO2 saccharide solution

### C. pH 7.04 buffered samples

### C1. Batch no NB01p57RO

### Non sterile

| Material | Weight fraction |
|---|---|
| Fructose | 52.0% |
| Glucose | 31.0% |
| 50 mMol Citric acid/NaOH buffer pH 7.04 | 17.0% |

### Description

### Non sterile base buffered saccharide solution.

### C2. Batch no NB01p57RO

### Sterile

| Material | Weight fraction |
|---|---|
| Fructose | 52.0% |
| Glucose | 31.0% |
| 50 mMol Citric acid/NaOH buffer pH 7.04 | 17.0% |

### Description

### Sterile base buffered saccharide solution

### C3. Batch no NB01p57RO1

### Non sterile

| Material | Weight fraction |
|---|---|
| Fructose | 52.0% |
| Glucose | 31.0% |
| 50 mMol Citric acid/NaOH buffer pH 7.04 | 17.0% |

### Description

### Non sterile base buffered RO1 saccharide solution.

### C4. Batch no NB01p57RO1

### Sterile

| Material | Weight fraction |
|---|---|
| Fructose | 52.0% |
| Glucose | 31.0% |
| 50 mMol Citric acid/NaOH buffer pH 7.04 | 17.0% |

### Description

### Sterile base buffered RO1saccharide solution

### C5. Batch no NB01p57RO2

### Non sterile

| Material | Weight fraction |
|---|---|
| Fructose | 52.0% |
| Glucose | 31.0% |
| 50 mMol Citric acid/NaOH buffer pH 7.04 | 17.0% |

### Description

### Non sterile base buffered RO2 saccharide solution.

### C6. Batch no NB01p57RO2

| Material | Weight fraction |
|---|---|
| Fructose | 52.0% |
| Glucose | 31.0% |
| 50 mMol Citric acid/NaOH buffer pH 7.04 | 17.0% |

### Description

### Sterile base buffered RO2 saccharide solution

### Example 2 - Efficacy of Synthetic honey compositions against planktonic MRSA (reference example)

MIC and MBC were assessed for the RO1 samples (containing 50 ppm glucose oxidase) and compared to Surgihoney^{™} (also containing 50 ppm glucose oxidase). See Andrews J. M. Journal of Antimicrobial Chemotherapy (2001) 48, suppl. S1, 5-16.

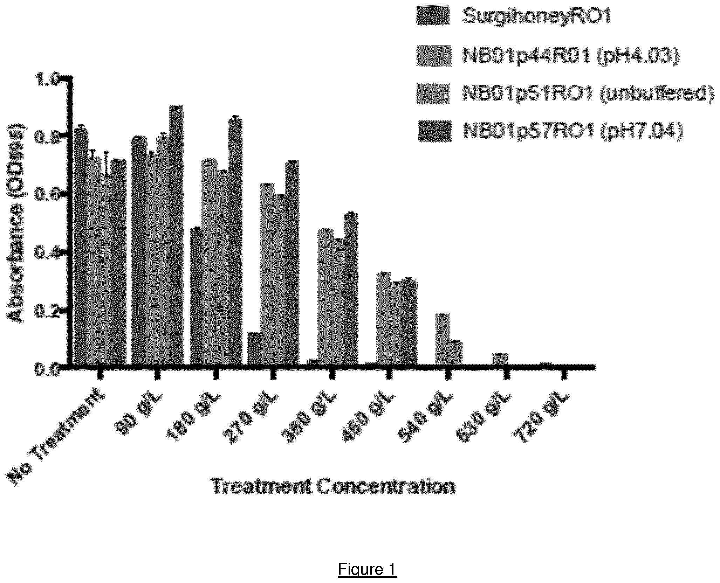

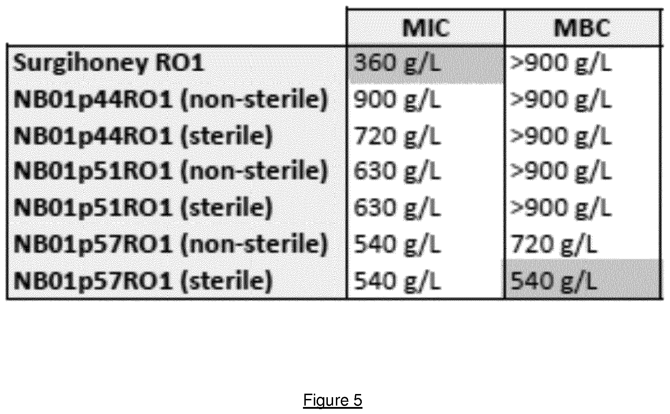

The results are shown in Figures 1 to 5.

The results show that, like Surgihoney, synthetic compositions containing glucose, glucose oxidase and fructose are able to inhibit microbial growth.

Out of all of synthetic compositions, the synthetic composition buffered at pH7.04 had the most effective MIC. Sterilised compositions were more effective than non-sterilised compositions, and synthetic composition buffered at pH7.04 synthetic had the most effective MBC when compared to other synthetic compositions and even when compared to Surgihoney.

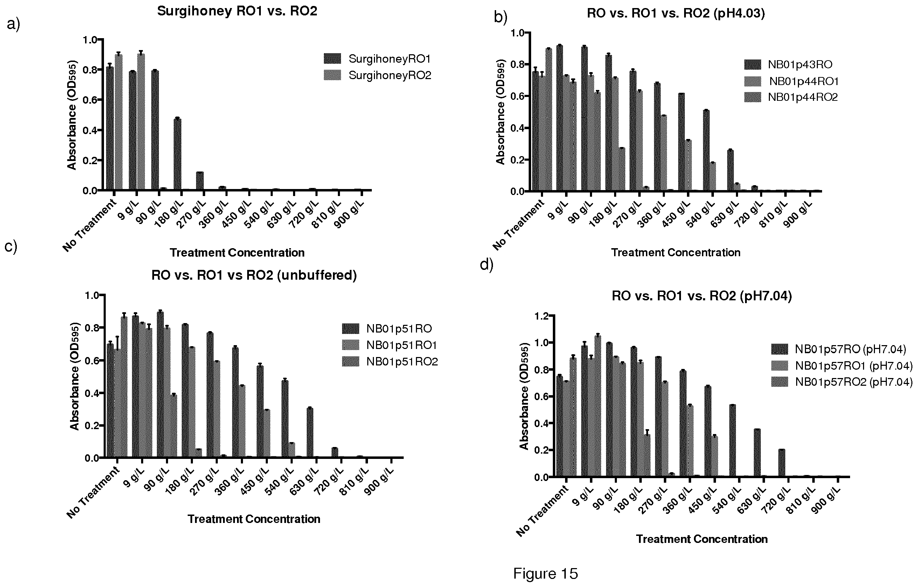

Figures 15 (a to d) and 16 show MIC and MBC results including SurgihoneyRO2 samples and synthetic RO2 samples.

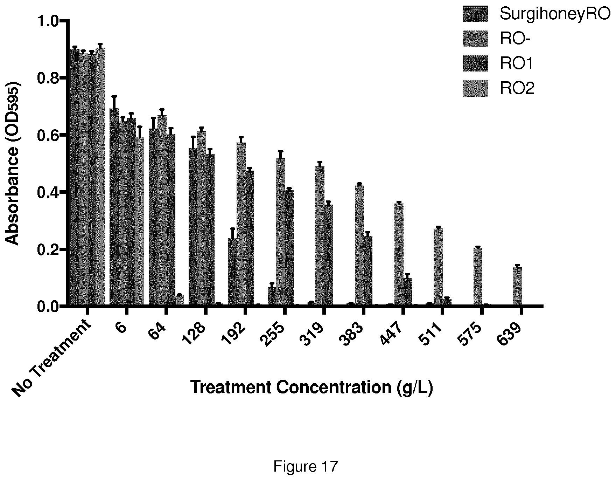

pH 7.04 formulations were tested against a planktonic MSSA isolate. Figure 17 shows the results obtained.

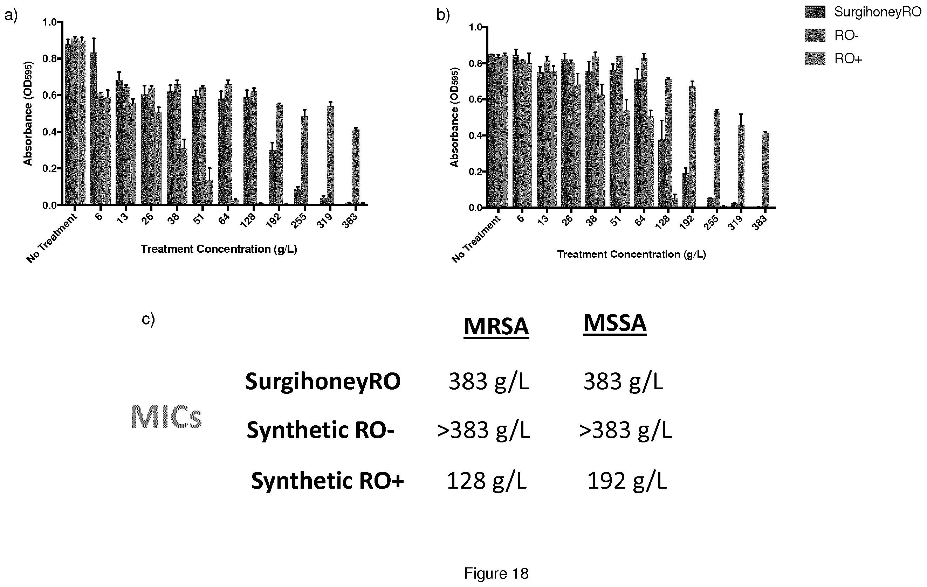

The synthetic RO2 composition was selected for further investigation. Figure 18 (a, b and c) show SyntheticRO (RO2; pH7.04) compared to SurgihoneyRO using planktonic phenotype. RO- indicates a product lacking enzyme activity.

### Example 3 -Surgihoney^{™} emulsions (reference example)

### Preparation

10g Surgihoney was dissolved in 10ml of glycerol. 10ml of paraffin oil was then added to a Rheometer (TA Instruments AR-G2) which had a Jacket Peltier and vane geometry attached. 1ml of PGPR (Polyglycerol polyricinoleate) was then added. The rheometer was then started under the following conditions; Shear rate 2000 1/s, Temperature set at 37.5° C. After 2 minutes, 10ml of Surgihoney-glycerol solution was added dropwise. Once a total of 10 minutes had elapsed the emulsion was transferred from the Jacket Peltier to a container.

### Optical Microscopy

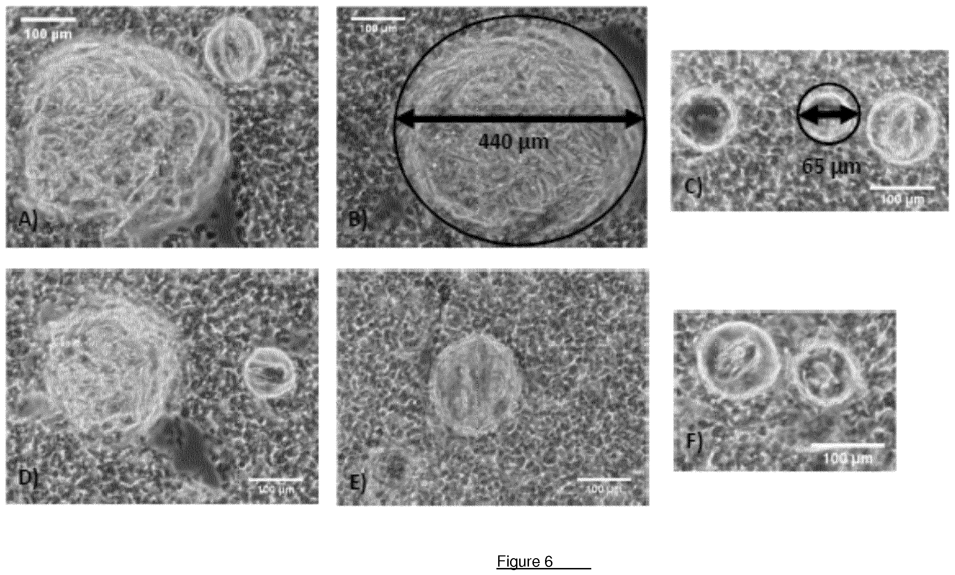

Optical microscopy revealed that the emulsion contained reverse micelles which encapsulated Surgihoney. Such micelles can be observed in Figure 6. The average micelle diameter was found to be 178 µm.

### Hydrogen peroxide tests

Hydrogen peroxide stick tests (Purchased from Sigma Aldrich (Quantofix^{®})) were used to detect hydrogen peroxide in the emulsion. The tests were carried out before and after addition of water, and showed that before addition of water, the emulsion produced no hydrogen peroxide, and after water was added, the emulsion tested positive for hydrogen peroxide. A positive test was indicated by a colour change to blue.

### Stability Test

The emulsion maintained its capacity to generate hydrogen peroxide after storage at ambient conditions for at least four weeks.

### Spray Test

The emulsion was added to a pump-action spray bottle and was found to be sprayable.

### Example 4 - Effects of different parameters on stability of Surgihoney^{™} emulsions (reference example)

The effects of changing the Surgihoney emulsion preparation method described in Example 3, one parameter at a time, were investigated. The changes and their effects are summarised below.

### i) Proportion of the oil phase to the Surgihoney-glycerol phase

Oil volumes greater than 10ml, and less than 10ml, were tested. The emulsion was found to be more stable when a lower volume of oil was used compared to the volume of the Surgihoney-glycerol phase. When the volume of the oil was less than 6ml, the emulsion was found to separate by less than 3% in total volume over 72 hours. A volume of 4ml allowed a separation of just 1.3% of the total volume over 72 hours. This stability is far greater than that of the method described in Example 3, which provided an emulsion with a separation of 9.4% of total volume over the same time period.

### ii) Volume of PGPR

PGPR volumes up to 4ml, and less than 1ml, were tested. The emulsion was more stable when a higher amount of PGPR was used. At a volume of 4ml, PGPR provided greater stability than with use of lower volumes, and far greater stability than that of the emulsion described in Example 3.

### iii) Shear rate

Shear rates from 1000 1/s to 3000 1/s were tested. The emulsion was more stable when a higher shear rate was applied. A shear rate of 3000 1/s produced the most stable emulsion. Separation of only 4.6% of the total volume over 72 hours was observed for emulsion prepared at this shear rate, compared with a separation volume of 9.4% of the total volume over the same time period for emulsion prepared as described in Example 3.

### iv) Temperature

Temperatures from 20°C to 40°C were tested. There was no noticeable trend regard the stability of the emulsions as temperature was increased. However a temperature of 40°C produced the most stable emulsion. Separation of only 3.1% of the total volume over 72 hours was observed for this emulsion.

### v) Length of shear

Shear times of 20 minutes and 30 minutes were tested, in addition to that used in the preparation method described in Example 3. However, there was no significant difference produced by extending the shear time.

### vi) Order of reagent addition

The effect of changing the order in which the reagents are added to the rheometer was tested. The effect of adding all of the components before starting the rheometer was compared with the effect of adding the Surgihoney-glycerol and oil components first, then adding the PGPR after 1-2 minutes. The most stable emulsion was formed when the PGPR was added last. The resulting emulsion provided a separation volume of 2.8% of the total volume over 120 hours.

### vii) Concentration of Surgihoney dissolved in glycerol

The following ratios of Surgihoney (g) to glycerol (ml) were tested: 1g:1ml; 0.5g:1ml; 2g:1ml. The ratio that produced the most stable emulsion was 1g:1ml, the same ratio used in the preparation method described in Example 3.

### viii) Sodium chloride

When sodium chloride is dissolved in the polar layer of the emulsion, it increases the polarity of this layer. It also forms electrostatic interactions with the lipid layer of the emulsion. The electrostatic interactions and increased polarity could improve stability and reduce coalescence. However, addition of sodium chloride (1g, 2g or 4g) was not found to influence the stability of the emulsion.

The effects of the changes are summarised in the table below:

| **Emulsion** | **SH (g)** | **Glycerol (ml)** | **Paraffin Oil (ml)** | **PGPR (ml)** | **Shear rate (1/s)** | **Temp. (⁰C)** | **Order of addition** | **Stability (% total vol. after 72 hrs)** |
|---|---|---|---|---|---|---|---|---|
| Ex 7 | 10 | 10 | 10 | 1 | 2000 | 37.5 | Oil, then PGPR, then SH/glycerol | 9.4 |
| Ex 8 (i) | 10 | 10 | **6** | 1 | 2000 | 37.5 | Oil, then PGPR, then SH/glycerol | 2.8 |
| Ex 8 (i) | 10 | 10 | **4** | 1 | 2000 | 37.5 | Oil, then PGPR, then SH/glycerol | 1.3 |
| Ex 8 (ii) | 10 | 10 | 10 | **4** | 2000 | 37.5 | Oil, then PGPR, then SH/glycerol | 2.7 |
| Ex 8 (iii) | 10 | 10 | 10 | 1 | **3000** | 37.5 | Oil, then PGPR, then SH/glycerol | 4.6 |
| Ex 8 (iv) | 10 | 10 | 10 | 1 | 2000 | **40.0** | Oil, then PGPR, then SH/glycerol | 3.1 |
| Ex 8 (vi) | 10 | 10 | 10 | 1 | 2000 | 37.5 | **SH/glycerol and oil, then PGPR** | 2.8* |

| | | | | | | | | |
|---|---|---|---|---|---|---|---|---|
| * (after 120 hrs) | | | | | | | | |

### Example 5 - Surgihoney^{™} emulsions with high stability (reference example)

The results from the changes described above were used to design a further method of preparing Surgihoney emulsion. This method is described below.

### Preparation

10g Surgihoney was dissolved in 10ml of glycerol. 4, 6, 8, or 10ml of Paraffin oil was then added to the rheometer (TA Instruments AR-G2) which had a Jacket Peltier and vane geometry attached. 10ml of Surgihoney-glycerol solution was then added to the rheometer. The rheometer was then started under the following conditions; Shear rate 3000 1/s, Temperature 40°C, gap 4000µm, Run time 10 minutes. After 1 minute 4ml of PGPR (Polyglycerol polyricinoleate) was then added. Once a total of 10 minutes had elapsed the emulsion was transferred from the rheometer to a container.

| Formulation number | Surgihoney-Glycerol (ratio - 1g:1ml) (ml) | PGPR (ml) | Paraffin oil (ml) | Total Volume Separation <11 Days (%) | Total Volume Separation after 20 days (%) |
|---|---|---|---|---|---|
| 1 | 10 | 4 | 10 | 0 | 0.7 |
| 2 | 10 | 4 | 4 | 0 | 0.9 |
| 3 | 10 | 4 | 4 | 0 | 1.0 |
| 4 | 10 | 4 | 4 | 0 | 1.6 |
| 5 | 10 | 4 | 6 | 0 | 1.2 |
| 6 | 10 | 4 | 8 | 0 | 0.9 |

All of the formulations were found to be highly stable, with a slight increase in stability observed as the volume of paraffin oil used was increased.

### Example 6 - Surgihoney^{™} Cream Formulation (reference example)

1.5g of Surgihoney was dissolved in 1.5ml of glycerol. 1g of sodium alginate was then dissolved in the Surgihoney-glycerol solution. Next 10ml of Paraffin oil was added to the Rheometer (TA Instruments AR-G2) which had a Jacket Peltier and vane geometry attached. 1ml of PGPR (Polyglycerol polyricinoleate) was then added. The rheometer was then started under the following conditions; Shear rate 2000 1/s, Temperature set at 37.5°C, gap 4000µm, Run time 10 minutes. After 2 minutes, 1.5ml of the Surgihoney-alginate and glycerol solution was added to the rheometer. After 3 minutes 8ml of calcium chloride solution was added dropwise to the rheometer. Once a total of 10 minutes had elapsed the emulsion was transferred from the Jacket Peltier to a container.

### Example 7 - Non-aqueous Surgihoney^{™} Cream Formulation (reference example)

The method described in Example 10 uses water to dissociate calcium chloride into its ions. This could potentially activate the Surgihoney to produce hydrogen peroxide, and limit the stability of the cream formulation. However, we have appreciated that calcium chloride can be dissociated using non-aqueous solvents, such as ethanol or acetic acid. We have also appreciated that glycerol is able to bind to free water. This property allows water to be used to dissolve the alginate, provided sufficient glycerol is present to prevent premature release of hydrogen peroxide.

The method described below uses ethanol as a solvent for calcium chloride, and glycerol to bind free water in the alginate solution.

1g of sodium alginate is dissolved in 15ml water. Next 30ml glycerol is added to the alginate solution and mixed. Then 30g Surgihoney is then dissolved in the solution. 10ml of Paraffin oil is then added to the rheometer (TA Instruments AR-G2) which has a Jacket Peltier and vane geometry attached. 10ml of Surgihoney solution is then added to the rheometer. The rheometer is then started under the following conditions: Shear rate 3000 1/s, Temperature 40°C, gap 4000µm, Run time 10 minutes. After 1 minute 4ml of PGPR (Polyglycerol polyricinoleate) is added. After 2 minutes 8ml of non-aqueous calcium chloride solution (1M Calcium chloride in ethanol) is added dropwise to the rheometer. Once a total of 10 minutes has elapsed, the emulsion is transferred from the rheometer to a container.

Summary of emulsion formulations described above.

| **Emulsio n/cream** | **SH (g)** | **Glycer ol (ml)** | **Paraffi n Oil (ml)** | **PGP R (ml)** | **NaAlg(g )/CaCl₂( ml)** | **Shea r rate (1/s)** | **Tem p. (⁰C)** | **Order of addition** |
|---|---|---|---|---|---|---|---|---|
| Ex 7 | 10 | 10 | 10 | 1 | - | 2000 | 37.5 | Oil, then PGPR, then SH/glycerol |
| Ex 9 | 10 | 10 | 4, 6, 8, or 10 | 4 | - | 3000 | 40.0 | Oil and SH/glycerol, then PGPR |
| Ex 10 | 1.5 | 1.5 | 10 | 1 | 1/8 (aq) | 2000 | 37.5 | Oil and PGPR, then SH/glycerol/Na Alg, then CaCl₂ |
| Ex 11 | 30 | 30 | 10 | 4 | 1/8 (non-aq) | 3000 | 40 | Oil and SH/glycerol/Na Alg(aq), then PGPR, then CaCl₂ (non-aq) |

Synthetic honey compositions of the invention could replace the Surgihoney in the Surgihoneybased emulsions and creams described herein.

### Example 8 - Treatment of Aphthous ulcers and Geographic tongue with Surgihoney^{™} (reference example)

Surgihoney was used to treat two patients with geographic tongue and recurrent aphthous ulcers. Surgihoney was applied topically and held over the affected areas three times a day for as long as possible before swallowing. This was continued for a week.

In both cases, the geographic tongue resolved in 48 hours. In one case, the ulcers did not progress, although this case had further aphthous ulcers 10 days after stopping treatment with Surgihoney. In the other case, the subject reported that the ulcers were not as bad as usual and no further ulcers were reported.

### Example 9 - Treatment with electron beam irradiation (reference example)

Samples of activated Acacia honey were prepared. Three glass sample vials were each filled with 10g of the activated honey. Each of the samples was tested by a Labtek PER100 peroxide test stick to determine hydrogen peroxide generation from the rate of colour generation. Samples were sterilised with electron beam irradiation at a dose of about 75 kGy and then tested for hydrogen peroxide generation.

Non Sterile Activated Acacia Honey Samples: Average measured rate of peroxide production 0.540 ppm/s standard Deviation 0.062.
Sterile Activated Acacia Honey Samples: Average measured rate of peroxide production 0.353 ppm/s standard Deviation 0.008

The honey samples maintained the ability to produce hydrogen peroxide after sterilisation with electron beam irradiation.

### Example 10 - Immunomodulatory effects of SurgihoneyRO Treatment on Epithelial Cells (reference example)

A human nasal epithelial cell line was used to investigate the immediate effect of SurgihoneyRO (also known as Surgihoney) on the integrity and function of the epithelium. In addition, a human mast cell line was used as a representative leukocyte to investigate immunological responses to SurgihoneyRO. Mast cells were selected because of their function as sentinel cells, being located just underneath the epithelium in tissues. Mast cells have close contact to the external environment, for example being found in the nasal mucosa, where they are ideally placed to participate in the early recognition of pathogens - by acting as immune effectors and modulatory cells with an essential role in linking innate and adaptive immunity in the host's defence against pathogens such as bacteria.

Another reason for using mast cells relates to findings of intracellular bacteria in mast cells (Hayes et al., J Allergy Clin Immunol 2015;135(6):1648-51). One of the objectives of this treatment would be to develop its use to target intracellular bacteria which act as a reservoir constantly seeding bacteria into the extracellular environment and therefore promoting ongoing inflammation and development of chronicity.

One of the objectives of the study was to determine whether treatment with SurgihoneyRO could elicit an immunomodulatory effect to the host. Figure 7 demonstrates the effects of SurgihoneyRO on the expression of four main gatekeeping genes for T cell differentiation. SurgihoneyRO treatment causes a shift in the Th balance toward Th₂ and Th₁₇ polarisation for nasal epithelial cells. The results show this as there is no observed significant increase in the expression of T-bet across the time course with either concentration of SurgihoneyRO. However there is a dose and time dependent increase in the expression of GATA3 this difference being significant at all time points (p≤0.05) when treated with 100g/L. At 2 hours, there is a significant increase in expression by 2.74 fold with 100g/L and 2.19 fold with 10g/L of SurgihoneyRO.

Figure 7 also shows that there is a dose and time dependent decrease in FOXP3 expression with significance at both 1 hour (0.34 fold decrease, p≤0.05) and 2 hours (0.60 fold decrease, p≤0.0001) when treated with 100g/L SurgihoneyRO. The nasal epithelial cells display an increase in the expression of the Th₁₇ gatekeeping gene RORγC. When treating with 100g/L SurgihoneyRO for 2 hours, the epithelial cells had a significant 1.89 fold increase in RORγC expression with p≤0.05. Data showed no effect of cell treatment with matching concentrations (10g/L and 100g/L) of the non-engineered base honey (Acacia) for all genes studied (data not shown).

The polarisation of Th₁₇ is further supported by the changes in gene expression of the Th₁₇ related cytokines interleukin 22 (IL22) and interleukin 23 receptor (IL23R) (Figure 8). There is a time and dose dependent increase in the expression of IL22; increasing from 0.22 to 2.05 fold (p≤0.05) and 1.67 (p≤0.01) across the time points with 100g/L and 0.86 fold to 1.33 and 1.38 (p≤0.05) with 10g/L respectively. Furthermore, there is a time and dose dependent increase in IL23R expression but only when treating with 100g/L; increasing from 2.74 fold (p≤0.001) to 8.54 fold (p≤0.05) and finally 14.28 fold (p≤0.05) across the time course.

Having illustrated the ability of SurgihoneyRO to influence Th₁₇ responses, we wanted to investigate whether the treatment had the capacity to mediate host responses to invading pathogens. Matrix metalloproteinases (MMPs) are known to be secreted by activated epithelial cells. In addition to matrix degrading and wound healing properties, MMPs also play an important role in host defence responses against infectious agents. MMP7 is involved in the activation of antimicrobial defensin, releasing mature TNF and chemokines to tackle infections. MMP9 can regulate immune responses and attract lymphocytic cell migration which facilitates tissue remodelling resulting from cleavage of extracellular matrix.

Figure 9a demonstrates a time and dose dependent upregulation of both MMP7 and MMP9 resulting in significant fold increases following treatments with both 10g/L and 100g/L of SurgihoneyRO. The expression of MMP7 significantly increased to a fold change of 4.56 (p≤0.01) with 10g/L and 8.04 (p≤0.05) with 100g/L at 2 hours. MMP9 also significantly increased to 1.37 fold (p≤0.01) with 10g/L and 3.73 fold (p≤0.05) with 100g/L. There were also significant increases in the expression of both defensin 5 and TNFα. There was a mean increase in defensin 5 expression of 0.9 fold (p≤0.01) and TNFα expression increased by 4.24 fold (p≤0.05) following 2 hours of treatment with 100g/L of SurgihoneyRO. This is direct evidence to support the hypothesis that SurgihoneyRO has the ability to boost the host response to invading pathogens (Figure 9a).

An essential function of the early innate immune response to pathogens is played by Toll-Like Receptors (TLRs) whose recognition of pathogen-associated microbial patterns (PAMPs), and endogenous danger-associated molecular patterns (DAMPs) initiates downstream signalling cascades resulting in the production of pro-inflammatory and effecter cytokines that have a direct effect on the adaptive immune response. Figure 9b shows an increase in the gene expression of two TLRs (TLR2 and TLR4) following the treatment of nasal epithelial cells with SurgihoneyRO (Figure 9b). There is a highly significant (p≤0.001) mean increase of 1.85 fold in expression of TLR2 when treating with 100g/L SurgihoneyRO at 2 hours.

We then studied the response of the mast cell line (HMC-1) to SurgihoneyRO treatment. In parallel to the effects seen with nasal epithelial cells, there is also an increase in the expression of Th₁₇ related cytokines following SurgihoneyRO treatment. When treating with 100g/L SurgihoneyRO for 2 hours, mast cells displayed significant increases of 1.89 fold in RORγC expression (p≤0.01), 1.35 fold in IL22 expression (p≤0.05) and 0.61 fold in IL23R expression (p≤0.05) respectively (Figure 10).

### Example 11 - Hydrogen Peroxide Production by SurgihoneyRO (reference example)

In addition to the potential immunomodulatory effects of SurgihoneyRO, we quantified the production of hydrogen peroxide by SurgihoneyRO for the concentrations used in this study (10g/L and 100g/L). The aim of this experiment was to validate whether the immunomodulatory effects observed previously were as a result of endogenous hydrogen peroxide production by SurgihoneyRO.

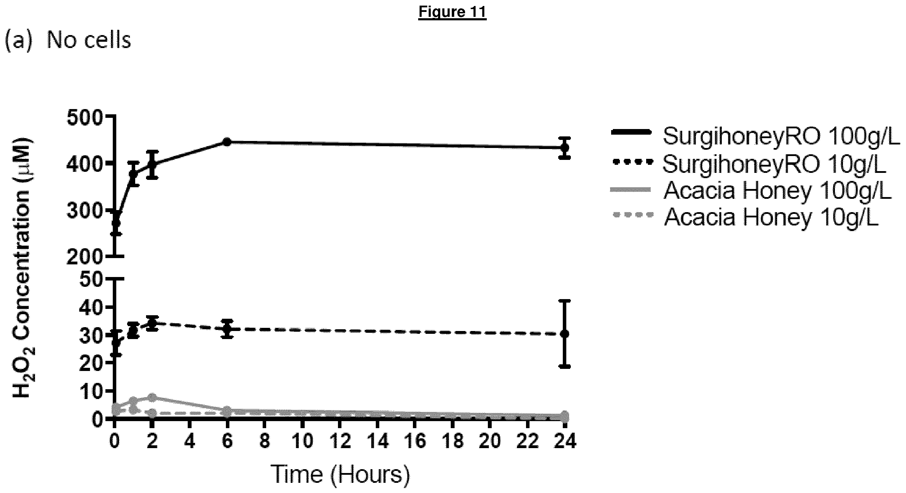

Figure 11a shows that the release of hydrogen peroxide by SurgihoneyRO is significantly higher than that of the non-engineered base honey (Acacia). The production of hydrogen peroxide peaks between 2 and 6 hours at which time it is roughly 12 fold higher for 100g/L than that for 10g/L. Following the exponential increase in the production, the level of hydrogen peroxide remains constant until 24 hours which was the last time point measured.

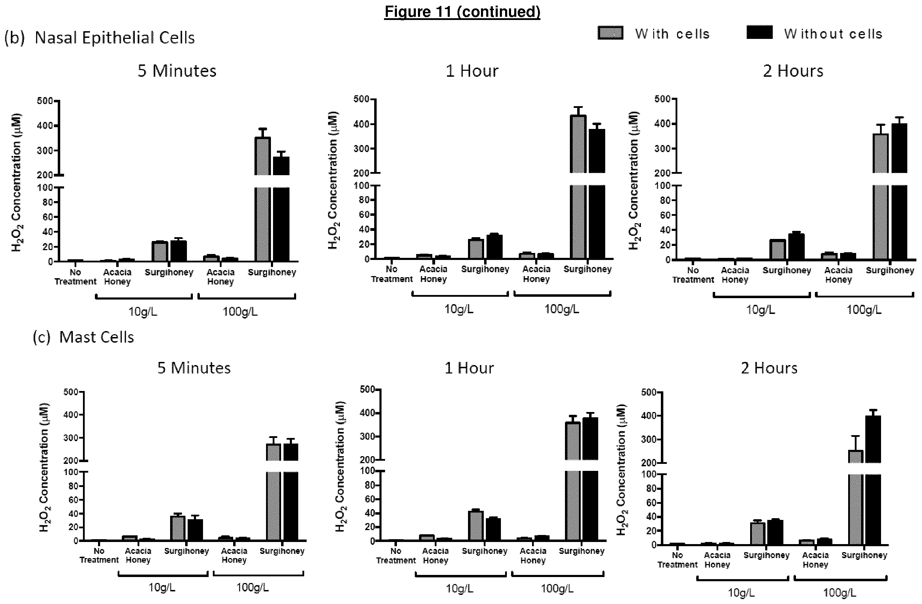

The study evaluated the production of hydrogen peroxide when the SurgihoneyRO and Acacia honey were used to treat both cell lines. It showed that the maximum amount of hydrogen peroxide produced by 100g/L SurgihoneyRO in the presence or absence of epithelial cells (Figure 11b) or mast cells (Figure 11c) was around 400 uM, peaking at 2 hours. There was no significant difference whether there are any cells present. The presence of cells within the culture made no significant difference suggesting that SurgihoneyRO is the main source of hydrogen peroxide in the cell culture.

### Example 12 - Immunomodulatory Effects of Exogenous Hydrogen Peroxide Treatment (reference example)

Having established the concentration of hydrogen peroxide production by SurgihoneyRO in Figure 11, we wanted to know whether the concentration of hydrogen peroxide produced in the cell culture is able to induce immunomodulatory changes. In this case, we treated cells with an appropriate concentration range of pure hydrogen peroxide, from 0 - 400uM.

The effect of the treatment with SurgihoneyRO correlated to that with a comparable concentration of pure hydrogen peroxide. Figure 12a shows that similar to the treatment with SurgihoneyRO, exogenous hydrogen peroxide elicits a dose dependent increase in GATA3 expression in nasal epithelial cells, with a significant fold increase of 0.92 (p≤0.5) when treating with 400µM hydrogen peroxide for 1 hour (Figure 12a).

A dose dependent increase in the expression of RORγC was also detected following exogenous hydrogen peroxide treatment, resulting in a mean increase in expression of 14.57 fold (p≤0.05) for the nasal epithelial cells and 2.83 fold (p≤0.05) for the mast cells with 400µM hydrogen peroxide.

These changes are also associated with increases of IL22 and IL23R for both cell lines (Figures 12b and 12c). With the nasal epithelial cells there is a steady increase in the expression of IL22 from 0.90 fold with 40µM to 1.42 fold with 200µM and 4.55 fold with 400µM (p≤0.001), and with IL23R the fold change in expression increases from 0.45 to 1.20 (p≤0.05) and 1.87 (p≤0.001) across the concentration range. With the mast cells there is an increase in expression of IL22 from 0.60 fold with 40µM to 1.25 with 200µM (p≤0.05) and 1.81 with 400µM (p≤0.01), and with IL23R the fold change in expression increases from 0.47 to 0.58 and 1.05 (p≤0.05) across the concentration range.

The treatments with exogenous hydrogen peroxide with epithelial cells and mast cells also caused a dose dependent increase in the expression of the anti-microbial MMP7 and MMP9 (Figures 13a and 13b). However there is only statistical significance with the expression of MMP9 for the nasal epithelial cells (1.47 fold with 400µM, p≤0.01) and MMP7 for the mast cells which have an increase of 3.07 fold (p≤0.05) and 3.31 fold (p≤0.05) with 200µM and 400µM respectively.

### Example 13 - A protective anti-inflammatory effect of SurgihoneyRO and Exogenous Hydrogen peroxide (reference example)

Exogenous hydrogen peroxide treatment can directly induce a protective anti-inflammatory effect on epithelial cells by increasing the expression of IL10. Figure 14a shows a significant increase in expression of the anti-inflammatory cytokine IL10 to 4.63 fold (p≤0.05) in the nasal epithelial cells treated with 400µM hydrogen peroxide. In concordance with these effects, we also demonstrated that there is a time dependent increase in the expression of the protective and anti-inflammatory cytokine IL10; increasing from a fold change of 0.01 to 2.65 (p≤0.05) and 4.73 (p≤0.05) across the time points with 100g/L and -0.81 to 3.01 (p≤0.05) and 4.85 with 10g/L respectively (Figure 14b). This increase in IL10 expression also correlated with the increase in GATA3 expression in the nasal epithelial cells supporting the shift towards the Th₂ lineage.

These data support a protective effect of both SurgihoneyRO and hydrogen peroxide and a shift in the balance towards the Th₂ lineage specifically for the nasal epithelial cells.

Taken together, these experiments clearly demonstrate an immunomodulatory effect of SurgihoneyRO treatment in both nasal epithelial and mast cells with a shift towards a Th₂ and Th₁₇ response, as well as anti-microbial and innate immunity responses. These effects could be mediated by the hydrogen peroxide production of SurgihoneyRO. It has been shown SurgihoneyRO has anti-inflammatory and wound healing effects on skin. In our study, we showed that these effects could be mediated through the upregulation of the anti-inflammatory cytokine IL10. These data shed new insights into the immunomodulatory properties of Surgihoney RO.

### Example 14 - Evaluation of Surgihoney^{™} and Synthetic Honey compositions on chronic rhinosinusitis-related mucosal bacterial strains of Staphylococcus aureus

SurgihoneyRO^{™} and synthetic RO (pH 7.04; RO2) were tested on both the *in vitro* planktonic phenotype and established biofilms of clinical MRSA and MSSA isolates. Biofilm viability was assessed by colony forming unit (cfu) enumeration and biomass assessed. by measurement of absorbance. Data were validated using confocal microscopy.

### • Materials and methods

### Bacterial strains and growth conditions

S. aureus strains were sub-cultured from frozen stocks onto Colombia blood agar (CBA) plates (Oxoid, UK) and incubated for 18 h at 37ºC and 5% CO₂, following which colonies were resuspended in Brain Heart Infusion (BHI) broth and grown to mid-exponential phase for experiments.

### Planktonic assays

Flat-bottomed 96-well plates (Fisher Scientific, UK) were inoculated with ~1.0 × 10⁶ planktonic bacteria per well (grown in supplemented BHI). SurgihoneyROTM and the synthetic products (RO+ and RO-) were prepared in BHI and added to wells at final concentrations of 6 g/L to 383 g/L. BHI alone was added in place of treatments for untreated controls. Cultures were incubated at 37oC and 5% CO₂ for 18 hours then turbidity measured by absorbance (OD595) using an EZRead 400 spectrophotometer (Biochrom; n=6).

### Biofilm assays

Mid-exponential planktonic cultures were used to inoculate individual wells of untreated 6-well polystyrene plates (~1.0 × 10⁸ planktonic bacteria per well; Corning Incorporated, USA). Cultures were incubated at 37°C and 5% CO₂ for 48h, replacing spent media with fresh BHI at 24 h. Prior to treatment spent media was removed and biofilms washed twice with Hanks' balanced salt solution (HBSS; Gibco, UK). Biofilms were treated with SurgihoneyRO, RO+ or RO- (all prepared in HBSS) at final concentrations of 7 to 142 g/L. HBSS alone was added in place of treatments for untreated controls. Biofilms were incubated at 37ºC and 5% CO2 for 24 h, following which the treatments were removed and biofilms washed twice with HBSS to remove unattached cells. Biofilms were resuspended in 1 ml HBSS by cell scraping and briefly vortexing, then serially diluted onto Columbia blood agar. Plates were incubated at 37°C and 5% CO₂ and colony forming units (CFUs) enumerated (n=5). To assess the total biofilm biomass 100µL of the resuspended biofilms was diluted 10-fold in BHI and the turbidity measured by absorbance (OD595) using a Jenway 6300 spectrophotometer.

### Confocal Microscopy

Mid-exponential planktonic cultures were used to inoculate 35 mm untreated glass-bottom CellView cell culture dishes (~1.0 × 108 planktonic bacteria per well; Greiner Bio One, UK). Cultures were incubated at 37°C and 5% CO₂ for 48 h,replacing spent media with fresh BHI at 24 h. Media was removed, biofilms washed twice with HBSS, then treated with 71 g/L RO+, 71 g/L RO-, or HBSS alone (untreated control) for 24 h at 37°C and 5% CO₂. Treatments wereremoved and biofilms washed twice with HBSS before staining with a LIVE/DEAD Baclight Bacterial Viability Kit (Life Technologies, UK) as per manufacturer's instructions. Biofilms were examined using an inverted Leica SP8 confocal microscope using a 63x oil immersion lens with sequential scanning of 2µm sections (Leica Microsystems, UK).

### Statistical analyses

Statistical analyses of in vitro planktonic and biofilm data were performed using one-way analysis of variance (ANOVA) and Kruskal-Wallis multiple comparisons tests. Comparative data with a P value of ≤0.05 were considered as statistically significant.

### • Results

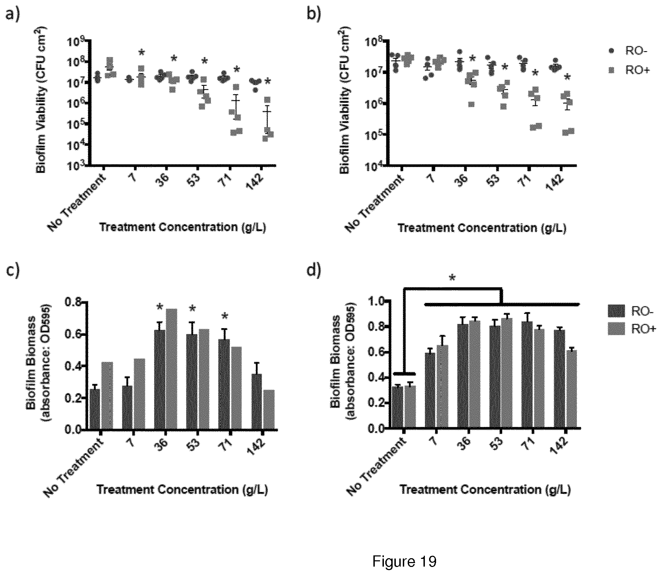

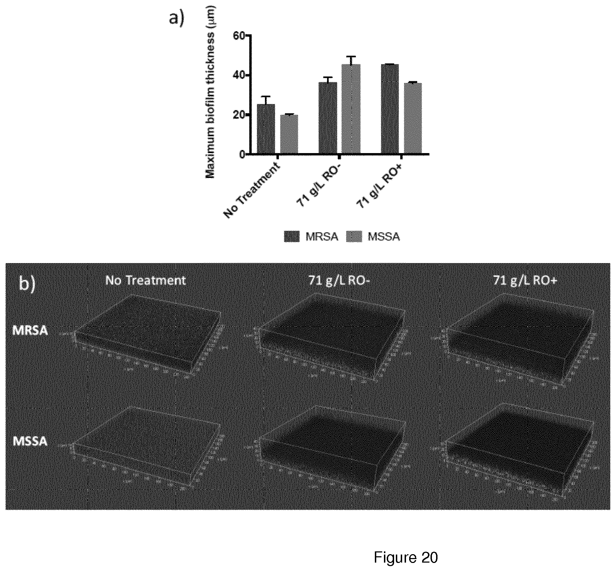

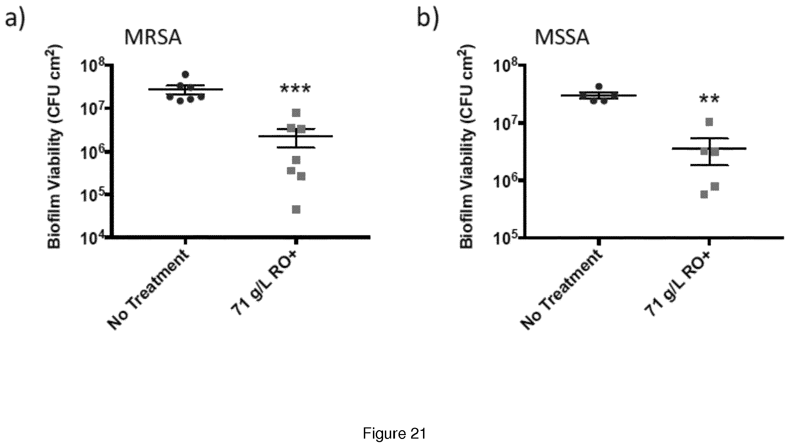

Treatment of established 48 h MRSA and MSSA biofilms using the same isolates revealed that treatment with the RO+ product for 24 h reduced biofilm viability in both instances (Figure 19 a&b). A log-fold reduction in viability was observed when treating MRSA biofilms with 53 and 71 g/L (p=0.0079), and a 2-log reduction when treating with 142 g/L (p=0.0159). In comparison, only a log-fold reduction was observed when treating MSSA biofilms with 36-142 g/L (p≤0.05). Treatment with RO- had no effect on viability of biofilms formed by either of the strains tested (Figure 19 a&b). Treatment of MRSA biofilms with either 36-71 g/L RO+ or RO- for 24 h also resulted in a significant increase in overall biofilm biomass (p≤0.05), whilst all concentrations tested (7-142 g/L) significantly increased the biomass of MSSA biofilms (p≤0.05). Confocal microscopy was used to validate the biofilm viability and biomass data. Both 48h established MRSA and MSSA biofilms demonstrated a reduction in viability and an increase in maximum biofilm thickness when treating with the 71 g/L RO- and RO+ products (Figures 20 a&b). The MRSA biofilm maximum height increased from 25.3 µm to 36.3 µm (RO-) and 45.3 µm (RO+), whilst the MSSA biofilm increased from 19.66 µm to 45.3 µm (RO-) and 35.99 µm (RO+). Finally, treatment of 48 h established biofilms formed by several clinical MRSA (n=7) and MSSA (n=5) isolates revealed that the viability of each biofilm was reduced with an average log-fold reduction observed within each group (Figure 21 a&b).

### Example 15 - Activity of Surgihoney^{™} against in vitro non-typeable Haemophilus influenza biofilms (reference example)

### • Materials and methods

### Bacterial strains and growth conditions

Bacterial strains used in this study were isolated from nasopharyngeal swabs of children aged 4 years. NTHi was sub-cultured from frozen stocks onto Colombia agar with chocolated horse blood (Oxoid, UK) and incubated for 18 h at 37°C and 5% CO₂, following which colonies were resuspended in Brain Heart Infusion (BHI) broth supplemented with 10 µg/ml Hemin and 2 µg/ml NAD, and grown to mid-exponential phase for experiments. Pseudomonas aeruginosa PA01 and a clinical methicillin resistant Staphylococcus aureus isolate were subcultured onto Colombia blood agar plates (Oxoid, UK) and grown in non-supplemented BHI. 65

### Planktonic assays

Flat-bottomed 96-well plates (Fisher Scientific, UK) were inoculated with ~1.0 ×10⁶ planktonic bacteria per well (grown in supplemented BHI). SurgihoneyRO^{™} and the non-engineered base honey(Acacia)were both prepared in supplemented BHI and added to wells at final concentrations of 6 g/L to 319 g/L. Supplemented BHI alone was added in place of treatments for untreated controls. Cultures were incubated at 37°C and 5% CO₂ for 18 hours then turbidity measured by absorbance (OD595) using an EZRead 400 spectrophotometer (Biochrom; n=6). 75

### Biofilm assays

Mid-exponential planktonic cultures were used to inoculate individual wells of untreated 6-well polystyrene plates (~1.0 × 10⁸ planktonic bacteria per well; Corning Incorporated, USA). Cultures were incubated at 37°C and 5% CO₂ for 48h, replacing spent media with fresh supplemented BHI (NTHi) at 24 h. Prior to treatment spent media was removed and biofilms washed twice with Hanks' balanced salt solution (HBSS; Gibco, UK). Biofilms were treated with SurgihoneyRO^{™} or Acacia (both prepared in HBSS) at final concentrations of 7 to 213 g/L. To assess the effect of pH biofilms were treated with HBSS adjusted to pH6.3 (the pH of 71 g/L SurgihoneyRO^{™} in HBSS). For adjuvant assays biofilms were treated with 300 µg/mL amoxicillin and 60 µg/mL clavulanic acid (Co-amoxiclav). HBSS alone was added in place of treatments for untreated controls. Biofilms were incubated at 37°C and 5% CO₂ for 2 h, following which the treatments were removed and biofilms washed twice with HBSS to remove unattached cells. Biofilms were resuspended in 1 ml HBSS by cell scraping and briefly vortexing the serially diluted onto Colombia agar with chocolated horse blood (NTHi). Plates were incubated at 37oC and 5% CO2 and colony forming units (c.f.u.) enumerated (n=4).

### Confocal Microscopy

Mid-exponential planktonic cultures were used to inoculate 35 mm untreated glass-bottom CellView cell culture dishes (~1.0 × 10⁸ planktonic bacteria per well; Greiner Bio One, UK). Cultures were incubated at 37°C and 5% CO₂ for 48 h, replacing spent media with fresh supplemented BHI at 24 h. Media was removed, biofilms washed twice with HBSS, then treated with 71 g/L SurgihoneyRO, 300/60 µg.ml co-amoxiclav or HBSS alone (untreated control) for 2 h at 37°C and 5% CO₂. Treatments were removed and biofilms washed twice with HBSS before staining with a LIVE/DEAD Baclight Bacterial Viability Kit (Life Technologies, UK) as per manufacturer's instructions. Biofilms were examined using an inverted Leica SP8 confocal microscope using a 63x oil immersion lens with sequential scanning of 2 µm sections (Leica Microsystems, UK).

### Hydrogen peroxide measurements

SurgihoneyRO^{™} and Acacia were prepared at a range of concentrations between 7 to 213 g/L in HBSS and incubated at 37°C and 5% CO₂ for 2 h. Hydrogen peroxide production was then measured using a Fluorimetric Hydrogen Peroxide Assay Kit (Sigma-Aldrich, UK) as per manufacturer's instructions using an untreated flat-bottomed black 96-well plate (Greiner BioOne, UK). In brief, a standard curve was generated using known concentrations of H₂O₂, to which SurgihoneyRO^{™} and Acacia samples were compared, including a negative HBSS control. A master mix comprised of red peroxidase, horseradish peroxidase and assay buffer was added to each standard and sample well. Samples were incubated at room temperature and protected from light for 30 minutes after which fluorescence was measured (excitation: 540 nm / emission: 590 nm; n=4).

### Statistical analyses

Statistical analyses of in vitro planktonic and biofilm data were performed using one-way analysis of variance (ANOVA) and Kruskal-Wallis multiple comparisons tests. Comparative data with a P value of ≤0.05 were considered as statistically significant.

### • Results

### SurgihoneyRO^{™} treatment reduces in vitro NTHi biofilm viability through increased H₂O₂ generation

Treatment of established 48 h in vitro biofilms with 71 and 142 g/L SurgihoneyRO^{™} for 2 hours resulted in a 4-log and 3-log reduction in viability respectively (P≤0.05), whilst treatment with 213 g/L reduced viability 5-log (P≤0.01 ; Fig. 22a). In comparison, treatment with the equivalent concentrations of Acacia had no effect on biofilm viability (P=0.75; Fig. 1b). A dosedependent increase in H₂O₂ levels in the surrounding media was also observed when treating with both Acacia and SurgihoneyRO^{™} (Fig. 22b). SurgihoneyRO^{™}, however, produced significantly higher levels of H₂O₂ at all concentrations tested, ranging from 10.7 - 71.2 µM in comparison with 0.24 - 6.5 µM generated when treating with the equivalent concentrations of Acacia. Furthermore, these data indicate that the minimum concentration of H₂O₂ effective in reducing NTHi biofilm viability, as evidenced with 71 g/l SurgihoneyRO^{™}, is approximately 14.2 to 25.7 µM (Fig. 22b). To account for a pH- mediated reduction in viability NTHi biofilms were also treated with HBSS adjusted to pH6.3, revealing no effect on biofilm viability (Fig. 22c).

### SurgihoneyRO^{™} is more effective than co-amoxiclav in the treatment of NTHi biofilms

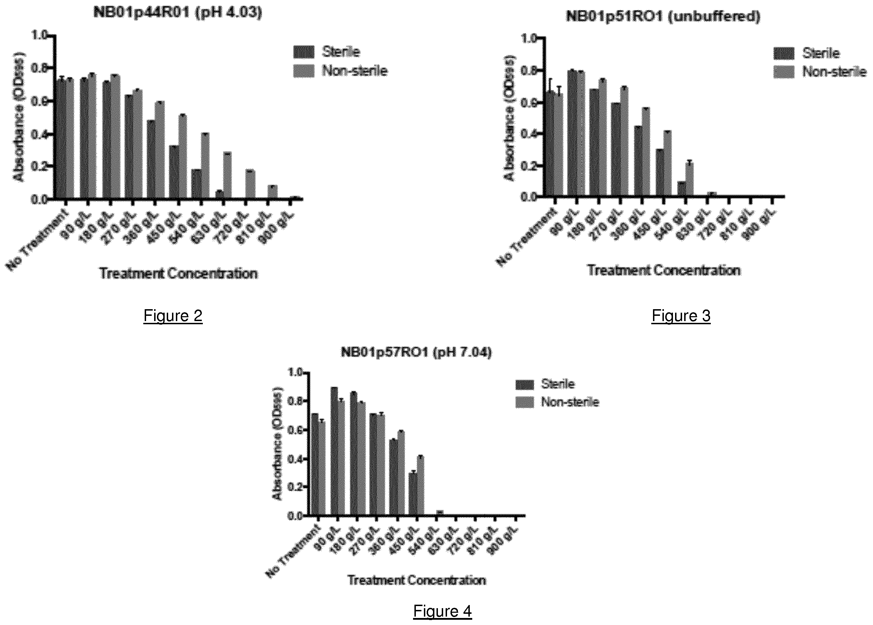

Following confirmation that 71 g/L SurgihoneyRO^{™} was effective in reducing NTHi biofilm viability the activity was compared to the conventional antibiotic co-amoxiclav, and also whether it could improve antibiotic efficacy when used as an adjuvant. Treatment of established 48 h in vitro biofilms with 71 g/L SurgihoneyRO^{™} for 2 hours resulted in a 5-log reduction in viability (P=0.029) whereas treatment with 300/60 µg.ml co-amoxiclav had no effect on viability (P=0.343; Fig. 2a). Combined treatment, however, did not improve co-amoxiclav efficacy (Fig. 23a). Confocal laser scanning microscopy confirmed the reduction in biofilm viability of 48h NTHi biofilms following treatment with 71 g/L SurgihoneyRO^{™} for 2 hours, and also the ineffectiveness of co-amoxiclav (Fig.2b-d). The confocal micrographs also demonstrated that SurgihoneyRO^{™} treatment had no obvious effect on overall biofilm biomass or ultrastructure, with all biofilms being ~70 µm in maximum height (Fig. 23b-d).

## Claims

1. A liquid or gel composition for generating antimicrobial activity, which comprises:
a purified enzyme that is able to convert a substrate to release hydrogen peroxide;
a purified substrate for the enzyme;
a solute in the form of a sugar having a solubility of at least 100g/100g water at 20°C and 1 atm, preferably at least 300g/100g water; and
a polymer,
wherein the composition has a water activity less than 0.6 and substantially no hydrogen peroxide, and
wherein the composition does not comprise honey.

2. A composition according to any preceding claim, wherein the solute is fructose.

3. A composition according to any preceding claim, comprising a buffer.

4. A composition according to any preceding claim, wherein the sugar has a solubility of at least 300g/100g water at 20°C and 1 atm.

5. A composition according to any preceding claim, wherein the enzyme is a glucose oxidase and the substrate for the enzyme is D-glucose.

6. A composition according to any preceding claim, which is sterile.

7. A composition according to any preceding claim, comprising substantially no:
i) zinc oxide; and/or
ii) catalase.

8. A composition according to any preceding claim, wherein the substrate is at least 90% pure and/or the enzyme is at least 90% pure and/or the solute is at least 90% pure.

9. A composition according to any preceding claim, comprising 25 to 2000 ppm of the enzyme, or 250 to 1500 ppm of the enzyme.

10. A composition according to any preceding claim, wherein the polymer comprises polyethylene glycol.

11. A composition according to any preceding claim, comprising substantially no peroxidase.

12. A composition according to any of claims 1 to 11, for use as a medicament, preferably for use in:
i) prevention, treatment, or amelioration of a microbial infection; or
ii) treatment of a wound; or
iii) treatment of a microbial infection that comprises a biofilm, optionally wherein the microbial infection comprises *Haemophilus influenza,* MRSA or MSSA; or
iv) treating chronic rhinosinusitis.

13. A wound dressing comprising a dressing material for dressing a wound, and a composition according to any of claims 1 to 11.

14. A method for producing a composition for generating antimicrobial activity, which comprises: contacting a purified enzyme that is able to convert a substrate to release hydrogen peroxide with a purified substrate for the enzyme, a solute in the form of a sugar, the solute having a solubility greater than 100g/100g water at 20°C and 1 atm, and a polymer,
wherein the composition has a water activity less than 0.6 and substantially no hydrogen peroxide, and wherein the composition does not comprise honey.

15. A method of sterilising a composition or wound dressing according to any of claims 1 to 11, or 13, which comprises exposing the composition or dressing to irradiation, preferably gamma irradiation or electron beam radiation.

16. A method of forming an antimicrobial solution comprising diluting a composition as defined in any of claims 1 to 11 in an aqueous solution such that there is sufficient free water to allow the enzyme to convert the substrate, preferably comprising diluting the composition to form a solution which contains 30 g/l to 150 g/l, 50 to 100 g/l or 65 to 75 g/l of the composition.

## Patentansprüche

1. Flüssige oder gelartige Zusammensetzung zur Erzeugung antimikrobieller Aktivität, die Folgendes umfasst:
ein gereinigtes Enzym, das ein Substrat umwandeln kann, um Wasserstoffperoxid freizusetzen;
ein gereinigtes Substrat für das Enzym;
einen gelösten Stoff in Form eines Zuckers mit einer Löslichkeit von mindestens 100 g/100 g Wasser bei 20°C und 1 atm, vorzugsweise mindestens 300 g/100 g Wasser; und
ein Polymer,
wobei die Zusammensetzung eine Wasseraktivität von weniger als 0,6 und im Wesentlichen kein Wasserstoffperoxid aufweist, und
wobei die Zusammensetzung keinen Honig enthält.

2. Zusammensetzung nach dem vorherigen Anspruch, wobei der gelöste Stoff Fructose ist.

3. Zusammensetzung nach einem vorherigen Anspruch, die einen Puffer umfasst.

4. Zusammensetzung nach einem vorherigen Anspruch, wobei der Zucker eine Löslichkeit von mindestens 300 g/100 g Wasser bei 20°C und 1 atm aufweist.

5. Zusammensetzung nach einem vorherigen Anspruch, wobei das Enzym eine Glucoseoxidase ist und das Substrat für das Enzym D-Glucose ist.

6. Zusammensetzung nach einem vorherigen Anspruch, die steril ist.

7. Zusammensetzung nach einem vorherigen Anspruch, die im Wesentlichen kein(e):
i) Zinkoxid; und/oder
ii) Katalase
enthält.

8. Zusammensetzung nach einem vorherigen Anspruch, wobei das Substrat zu mindestens 90% rein ist und/oder das Enzym zu mindestens 90% rein ist und/oder der gelöste Stoff zu mindestens 90% rein ist.

9. Zusammensetzung nach einem vorherigen Anspruch, die 25 bis 2000 ppm des Enzyms oder 250 bis 1500 ppm des Enzyms umfasst.

10. Zusammensetzung nach einem vorherigen Anspruch, wobei das Polymer Polyethylenglykol umfasst.

11. Zusammensetzung nach einem vorherigen Anspruch, die im Wesentlichen keine Peroxidase enthält.

12. Zusammensetzung nach einem der Ansprüche 1 bis 11 zur Verwendung als Arzneimittel, vorzugsweise zur Verwendung bei:
i) Vorbeugung, Behandlung oder Milderung einer mikrobiellen Infektion; oder
ii) Behandlung einer Wunde; oder
iii) Behandlung einer mikrobiellen Infektion, die einen Biofilm umfasst, wobei die mikrobielle Infektion optional *Haemophilus influenza,* MRSA oder MSSA umfasst; oder
iv) Behandlung von chronischer Rhinosinusitis.

13. Wundverband, der ein Verbandsmaterial zum Verbinden einer Wunde und eine Zusammensetzung nach einem der Ansprüche 1 bis 11 umfasst.

14. Verfahren zur Herstellung einer Zusammensetzung zur Erzeugung antimikrobieller Aktivität, das Folgendes beinhaltet: Inkontaktbringen eines gereinigten Enzyms, das ein Substrat umwandeln kann, um Wasserstoffperoxid freizusetzen, mit einem gereinigten Substrat für das Enzym, einem gelösten Stoff in Form eines Zuckers, wobei der gelöste Stoff eine Löslichkeit von mehr als 100 g/100 g Wasser bei 20°C und 1 atm aufweist, und einem Polymer,
wobei die Zusammensetzung eine Wasseraktivität von weniger als 0,6 und im Wesentlichen kein Wasserstoffperoxid aufweist, und wobei die Zusammensetzung keinen Honig enthält.

15. Verfahren zum Sterilisieren einer Zusammensetzung oder eines Wundverbands nach einem der Ansprüche 1 bis 11 oder 13, das das Bestrahlen der Zusammensetzung oder des Verbands vorzugsweise mit Gammastrahlen oder Elektronenstrahlen beinhaltet.

16. Verfahren zum Bilden einer antimikrobiellen Lösung, das das Verdünnen einer Zusammensetzung nach einem der Ansprüche 1 bis 11 in einer wässrigen Lösung beinhaltet, so dass ausreichend freies Wasser vorhanden ist, um dem Enzym die Umwandlung des Substrats zu ermöglichen, das vorzugsweise das Verdünnen der Zusammensetzung zur Bildung einer Lösung beinhaltet, die 30 g/l bis 150 g/l, 50 bis 100 g/l oder 65 bis 75 g/l der Zusammensetzung enthält.

## Revendications

1. Composition liquide ou en gel pour produire une activité antimicrobienne, laquelle comprend:
une enzyme purifiée qui est capable de convertir un substrat pour libérer du peroxyde d'hydrogène;
un substrat purifié pour l'enzyme;
un soluté sous la forme d'un sucre ayant une solubilité d'au moins 100 g/100 g d'eau à 20 °C et 1 atm, de préférence d'au moins 300 g/100 g d'eau; et
un polymère,
où la composition a une activité d'eau de moins de 0,6 et sensiblement pas de peroxyde d'hydrogène, et
où la composition ne comprend pas de miel.

2. Composition selon l'une quelconque des revendications précédentes, dans laquelle le soluté est du fructose.

3. Composition selon l'une quelconque des revendications précédentes, comprenant un tampon.

4. Composition selon l'une quelconque des revendications précédentes, dans laquelle le sucre a une solubilité d'au moins 300 g/100 g d'eau à 20 °C et 1 atm.

5. Composition selon l'une quelconque des revendications précédentes, dans laquelle l'enzyme est une glucose oxydase et le substrat pour l'enzyme est du D-glucose.

6. Composition selon l'une quelconque des revendications précédentes, qui est stérile.

7. Composition selon l'une quelconque des revendications précédentes, ne comprenant sensiblement pas:
i) d'oxyde de zinc; et/ou
ii) de catalase.

8. Composition selon l'une quelconque des revendications précédentes, dans laquelle le substrat est d'au moins 90 % pur et/ou l'enzyme est d'au moins 90 % pure et/ou le soluté est d'au moins 90 % pur.

9. Composition selon l'une quelconque des revendications précédentes, comprenant de 25 à 2000 ppm de l'enzyme, ou de 250 à 1500 ppm de l'enzyme.

10. Composition selon l'une quelconque des revendications précédentes, dans laquelle le polymère comprend du polyéthylène glycol.

11. Composition selon l'une quelconque des revendications précédentes, ne comprenant sensiblement pas de peroxydase.

12. Composition selon l'une quelconque des revendications 1 à 11, pour une utilisation en tant que médicament, de préférence pour une utilisation dans:
i) la prévention, le traitement ou l'amélioration d'une infection microbienne; ou
ii) le traitement d'une plaie; ou
iii) le traitement d'une infection microbienne qui comprend un biofilm, optionnellement où l'infection microbienne comprend *Haemophilus influenza,* SARM ou SAMS; ou
iv) le traitement d'une rhinosinusite chronique.

13. Pansement pour plaie comprenant une matière de pansement pour panser une plaie, et une composition selon l'une quelconque des revendications 1 à 11.

14. Procédé de production d'une composition pour produire une activité antimicrobienne, qui comprend: mettre une enzyme purifiée qui est capable de convertir un substrat pour libérer du peroxyde d'hydrogène en contact avec une substrat purifié pour l'enzyme, un soluté sous la forme d'un sucre, le soluté ayant une solubilité supérieure à 100 g/100 g d'eau à 20 °C et 1 atm, et un polymère,
dans lequel la composition a une activité d'eau de moins de 0,6 et sensiblement pas de peroxyde d'hydrogène, et dans lequel la composition ne comprend pas de miel.

15. Procédé de stérilisation d'une composition ou d'un pansement pour plaie selon l'une quelconque des revendications 1 à 11 ou 13, qui comprend exposer la composition ou le pansement à l'irradiation, de préférence à l'irradiation gamma ou à un rayonnement de faisceau d'électrons.

16. Procédé de préparation d'une solution antimicrobienne comprenant diluer une composition selon l'une quelconque des revendications 1 à 11 dans une solution aqueuse de sorte qu'il y a suffisamment d'eau libre pour permettre à l'enzyme de convertir le substrat, comprenant de préférence diluer la composition pour former une solution qui contient de 30 g/l à 150 g/l, de 50 à 100 g/l ou de 65 à 75 g/l de la composition.
